(19) Europäisches Patentamt
European Patent Office
Office européen des brevets

(11) EP 0 269 947 B2

(12) NEW EUROPEAN PATENT SPECIFICATION

(45) Date of publication and mention of the opposition decision:
**16.10.1996 Bulletin 1996/42**

(45) Mention of the grant of the patent:
**22.07.1992 Bulletin 1992/30**

(21) Application number: **87116996.7**

(22) Date of filing: **17.11.1987**

(51) Int Cl.[6]: **C07F 9/547**, A61K 31/675

# (54) Antiviral phosphonomethoxyalkylene purine and pyrimidine derivatives

Antiviralen Phosphonomethoxyalkylen-Purin- und -Pyrimidin-Derivate

Dérivés de phosphonométhoxyalkylène-purine et pyrimidine antiviraux

(84) Designated Contracting States:
**AT BE CH DE ES FR GB GR IT LI LU NL SE**

(30) Priority: **18.11.1986 US 932112**
**04.11.1987 US 114340**

(43) Date of publication of application:
**08.06.1988 Bulletin 1988/23**

(73) Proprietors:
- **INSTITUTE OF ORGANIC CHEMISTRY AND BIOCHEMISTRY OF THE ACADEMY OF SCIENCES OF THE CZECH REPUBLIC**
  **CS-166 10 Praha 6 (CS)**
- **Rega Stichting Vzw.**
  **B- 3000 Leuven (BE)**

(72) Inventors:
- **Webb, Robert R., II**
  **Guilford Connecticut 06437 (US)**
- **Bronson, Joanne J.**
  **Madison Connecticut 06443 (US)**
- **Martin, John C.**
  **Cheshire Connecticut 06410 (US)**

(74) Representative: **Barz, Peter, Dr. et al**
**Patentanwalt**
**Kaiserplatz 2**
**80803 München (DE)**

(56) References cited:

| | |
|---|---|
| **EP-A- 253 412** | **EP-A- 0 205 826** |
| **EP-A- 0 206 459** | **EP-A- 0 253 412** |
| **AU-A- 5 632 886** | **AU-A- 5 646 886** |

- **Ernst Juckner ; "Fortschritte der Arzneimittelforschung" vol.7 pages 306/307 (1964)**
- **Alfred Burger; "Medical Chemistry" third edition part 1 pages 74/75 (1979)**

Remarks:
The file contains technical information submitted after the application was filed and not included in this specification



Printed by Jouve, 75001 PARIS (FR)

## Description

Field of the Invention

This invention concerns nucleotide analogs and their compositions and use. In particular it concerns acyclic phosphonomethoxyalkylene derivatives of purine and pyrimidine bases.

Background of the Invention

Infectious viral diseases are recognized as an important medical problem. Progress against infectious viral diseases requires the development of drugs with selective antiviral activity while remaining benign to normal cell lines. A number of antiviral agents currently under study which seem to possess some selectivity, are nucleoside analogs. In general, these compounds are structural analogs of the naturally occurring nucleosides. Structural modification in either the purine or pyrimidine base nucleus and/or the saccharide component results in a synthetically modified nucleoside derivative which, when incorporated into a viral nucleic acid forming process, acts to disrupt further synthesis of viral nucleic acid. Effectiveness of these antiviral agents depends on selective conversion of viral enzymes, but not by host enzymes, to the corresponding nucleotide analog which is then converted to the triphosphate and incorporation into viral nucleic acid occurs. A problem with this antiviral strategy has been the emergency of certain viral strains whose enzymes poorly promote phosphorylation of the nucleoside analogs. To circumvent this problem,intact nucleotide analogs appear to be potentially quite useful as antivirals for incorporation into viral nucleic acid.

Reist and Sturm in PCT/US 84/04748, published December 6, 1984, disclosed new phosphonic acid analogs of nucleoside phosphates which are useful as antivirals for incorporation into viral DNA. The structural formula for these compounds is shown below as 1.

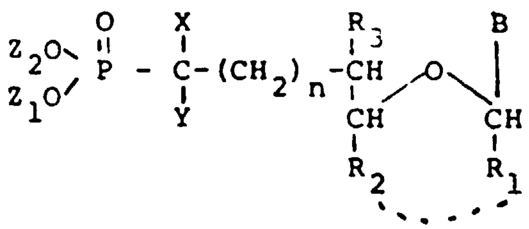

1

In the Reist compounds, B is a purine or pyrimidine base; $R_1$ and $R_2$ together complete a β-pentofuranose sugar or $R_1$ is H and $R_2$ is H or hydroxymethyl; $R_3$ is H or OH; X is H, OH or together with Y is carbonyl oxygen and Y can also be H; $Z_1$ and $Z_2$ are H or alkyl. These art compounds are generally distinguished from the compounds of the instant invention by 1) the ether-oxygen link to the carbon atom attached to the base which is intended to preserve or mimic the acetal oxygen bond of a pentofuranose sugar ring; and 2) the phosphate modification is a phosphonoalkylene moiety. In contrast, the acyclic sugar analog component of the instant compounds is comprised of an all carbon atom backbone up to a phosphonomethoxy moiety.

Similarly, synthesis and anti-Herpes-Virus activity of phosphate and phosphonate derivatives of 9-[(1,3-dihydroxy-2-propoxy)methyl]guanine (Formula 2) was disclosed by Prisbe, et al., in J. Med. Chem., 1986, 29, 671.

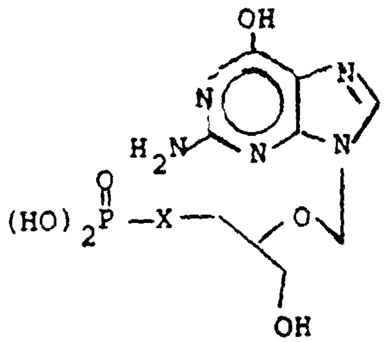

(X=O, $CH_2$)

2

More closely related are adenine phosphonic acid analogs (Formula 3) and their syntheses which were disclosed in the UK Patent Application of Holy, et al., GB 2,134,907A, published 8/22/84.

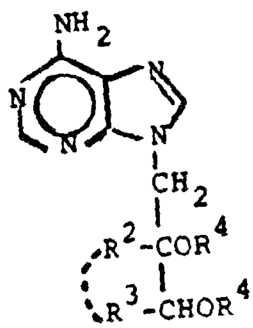

3

In formula 3, $R_2$ and $R_3$ are H or together complete a ribonucleoside ring; and both $R_4$ are alternately a hydrogen and $-CH_2P(O)(OH)_2$ group.

A preferred example of one of these compounds, known as (S)-HPMPA (Formula 4) was disclosed by DeClercq, et al., in Nature, 1986, 323,pp. 464-467 and earlier by Holy, et al., Nucleic Acids Research, Symposium Series No. 14, 1984 pp. 277-278.

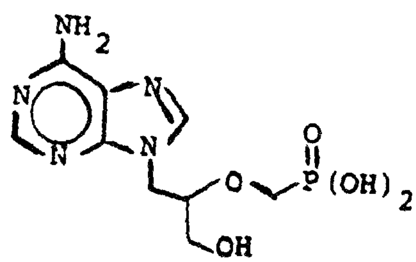

4

EP-A- 253 412 which is a prior art document according to Article 54 (3) EPC discloses N-phosphonylmethoxyalkyl derivatives of pyrimide und purine bases of the general formula

$$B-CH_2\underset{R}{C}H-OCH_2-P(O)(OH)_2 \quad (I).$$

wherein R is a hydrogen atom or a hydroxymethyl group and B is an optionally substituted pyrimidinl-yl, pyrimidin-3-yl, purin-3-yl. purin-7-yl or purin-9-yl residue, whereby unsubstituted adenin-9-yl is excluded, and the salts thereof with alkali metals, ammonia and amines.

These compounds exhibit antiviral activity.

AU-A-56468/86 discloses therapeutic compositions for use in the treatment of virus diseases which comprise as an active ingredient the phosphonylmethoxyalkyl adinine of the general formula:

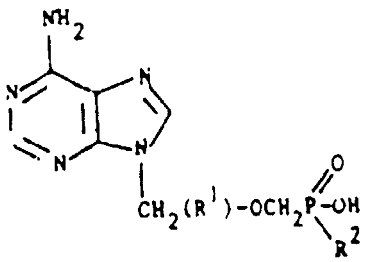

(1)

wherein $R^1$ is methylene, $-CH(OH)-CH_2-$ or $-CH-CH_2OH$ and $R^2$ is a hydroxyl group, and, wherein, when $R^1$ is different from methylene, $R^1$ and $R^2$ may be linked with each other to form a cyclic ester group, said adenine derivative being in (RS) or (S) form when R' is different from methylene and further being in the form of a free acid or a salt thereof.

AU-A-56328/86 discloses 9-(phosphonylmethoxyalkyl)adenines of the general formula:

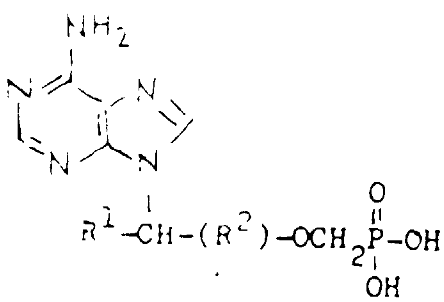

These compounds are antiviral drugs or may be converted to such drugs.

The present invention relates to phosphonomethoxyalkylene purine and pyrimidine derivatives which have been synthesized and found to possess useful antiviral activity. These compounds differ from the natural nucleotides by having structural variations in their sugar analog component which can be accompanied by variation in their nucleotide base moiety also. Additionally these compounds differ from the naturally occurring phosphate structure of nucleotides by nature of the oxygen-carbon-phosphorous bonds in these phosphonomethoxy derivatives. The compounds of this invention are represented by structural formula I.

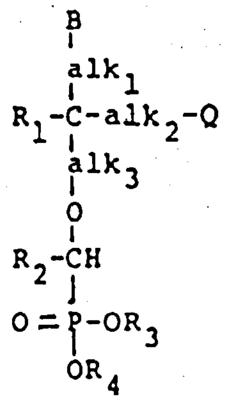

I

wherein B is a purine or pyrimidine base selected from the group consisting of xanthine, hypoxanthine, guanine, 8-bromoguanine, 8-chloroguanine, 8-aminoguanine, 8-hydrazinoguanine, 8-hydroxyguanine, 8-methylguanine, 8-thioguanine, 2-aminopurine, 2,6-diaminopurine, cytosine, 5-ethylcytosine, 5-methylcytosine, thymine, uracil, 5-bromouracil, 5-ioduracil, 5-ethyluracil, 5-propyluracil, 5-vinyluracil, and 5-bromovinyluracil:

$alk_1$, $alk_2$ and $alk_3$ are independently selected from a chemical bond or $C_1$-$C_4$ alkylene;
Q is hydrogen or hydroxyl;
$R_1$ and $R_2$ are independently selected from hydrogen and $C_1$-$C_4$ alkyl; and $R_3$ and $R_4$ are independently selected from hydrogen, $C_1$-$C_6$ alkyl, phenyl and phenyl-$C_1$-$C_4$ alkylene;
and the corresponding salts, zwitterions, and/or solvates except those compounds wherein $alk_1$ is methylene, $R_1$ is hydrogen, $alk_2$ is a chemical bond and Q is hydrogen, $alk_3$ is a chemical bond, $R_2$, $R_3$ and $R_4$ are hydrogen and B is uracil-1-yl, thymine-1-yl, cytosine-1-yl, guanine-9-yl, or hypoxanthine-9-yl, and
except those compounds wherein $alk_1$ is methylene, $R_1$ is hydrogen, $alk_2$ ist methylene and Q is hydroxyl, $alk_3$ is a chemical bond, $R_2$, $R_3$ and $R_4$ are hydrogen, and B is uracil-1-yl, cytosine-1-yl, thymine-1-yl, guanine-9-yl, guanine-7-yl, hypoxanthine-9-yl, or 2-aminopurine-9-yl, and except (RS) compounds wherein $alk_1$ and $alk_2$ are methylene, $alk_3$ is a chemical bond, Q is hydroxyl, $R_1$, $R_2$, $R_3$ and $R_4$ are hydrogen and B is 2,6-diaminopurine-9-yl, and except compounds having the formula:

$$B\text{-}CH_2CH_2OCH_2P(O)\,(OC_2H_5)_2,$$

wherein B is xanthine, hypoxanthine, guanine, 2-amino-purine-9-yl, 2,6-diaminopurine-9-yl, cytosine, thymine or uracil.

Compounds of the instant invention are also to include the corresponding salts, which may be base salts of the phosphonic acid moiety or an acid addition salt of the heterocyclic base; in addition to the zwitterionic forms and/or solvates of compounds for Formula I.

The compounds of the present invention can exist as optical isomers and both racemic and diastereomeric mixtures of these isomers which may exist for certain compounds as well as the individual optical isomers which are all within the scope of the present invention. While the racemic mixtures can be separated into their individual isomers through well-known techniques such as, for example, the separation of diastereomeric salts formed with optically active adjuncts, e.g. acids or bases followed by conversion back to the optically active substrates; in most instances, for compounds of the present invention, the preferred optical isomer can be synthesized by means of stereospecific reactions, beginning with the appropriate stereoisomer of the desired starting material. As indicated, the present invention also pertains to the pharmaceutically acceptable non-toxic salts of these compounds. Such salts may include those derived by combination of appropriate cations such as alkali and alkaline earth metal ions or ammonium and quaternary amino ions with the acid anion moiety of the phosphonic acid group. In addition salts may be formed from acid addition of certain organic and inorganic acids with basic centers of the purine, specifically guanine, or pyrimidine base. Finally it is to be understood that compounds of the present invention in their un-ionized as well as zwitterionic form and/or in the form of solvates are also considered part of the present invention.

Compounds of the present invention also exist in subclasses: two broad subclasses being those wherein B is either a purine or a pyrimidine base. Of these broad subclasses there are preferred classes wherein the purine base is a guanine or a substituted guanine moiety and where the pyrimidine bases are either thymine or cytosine. The most preferred class of compounds is that wherein B is guanine or substituted guanine.

Preferred classes of sugar analog components, e.g.

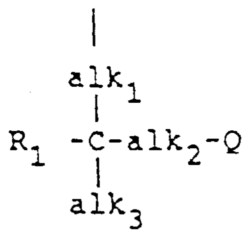

are those wherein $alk_2$ is a chemical bond and Q is hydrogen and those wherein $alk_2$ is methylene and Q is hydroxyl.

Compounds of the present invention may also be subclassed according to the structure of the phosphonate moiety. These classes are comprised of the diester, the monoester, and the diacid. Preferred subclasses of the phosphonate moiety are the monoester and the diacid.

The compounds of this invention can be prepared by the following two general procedures. The compounds wherein Q is hydrogen and $alk_2$ is a chemical bond can be generally prepared by Synthetic Scheme I and those compounds wherein Q is hydroxyl can generally be prepared from Synthetic Scheme II.

Synthetic Scheme I

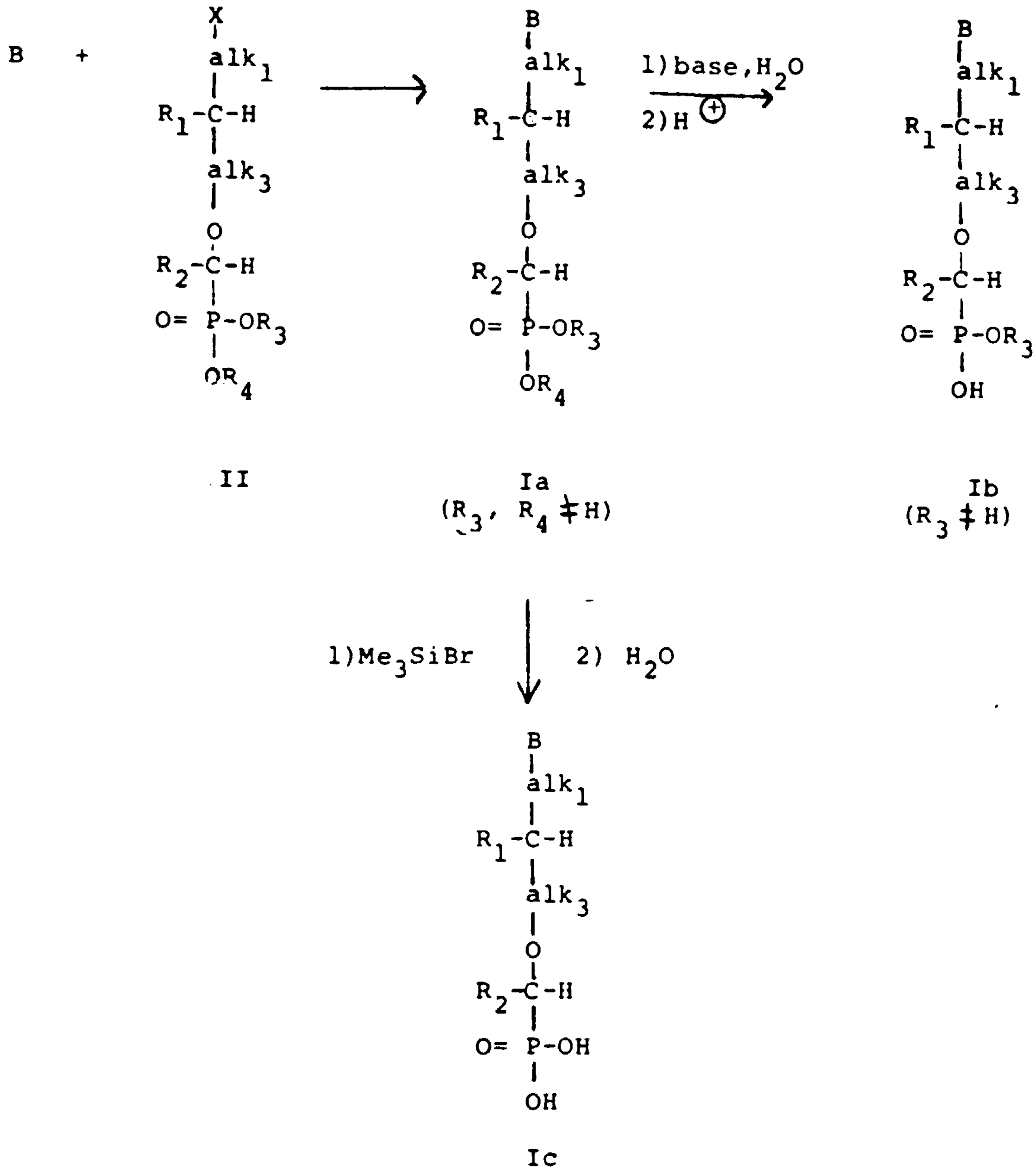


II

Ia ($R_3$, $R_4 \neq H$)

Ib ($R_3 \neq H$)

Ic

In Scheme I, B, $alk_1$, $alk_3$, $R_1$, $R_2$, $R_3$ and $R_4$ are as defined herein above. The symbol X represents a standard organic synthetic leaving group moiety such as chloride, bromide, iodide, tosylate, mesylate, triflate and the like. It is understood that in Scheme I, $alk_2$ is a chemical bond and Q is hydrogen. In the sequence of reactions comprising Scheme I the base B is converted to an anion by treatment with a base, such as an alkali metal hydride, in a non-reactive solvent, such as dimethylformamide (DMF), by stirring together for about 1 to 3 hours while in the temperature range of from room temperature to about 130°. The base anion is alkylated with a phosphonate diester intermediate of Formula II to give the diester product of Formula Ia. This diester may be converted either to the monoester, Ib or the diacid, Ic.

The conversion of the diester Ia to the monoester Ib can be accomplished either by dissolving Ia in aqueous hydroxide solution and holding at a temp between room temperature and 80° for about 1 to 6 hrs. Alternatively, when the base has an acid-labile protecting group on a reactive ring moiety of the base, the conversion of Ia to Ib, with concomitant removal of the protecting group, proceeds by dissolving the protected Ia compound in dilute acid, such as HCl, and holding in the temperature range from about room temperature to about 100° for about 1 to 6 hours.

The conversion of the diester Ia to the diacid Ic is readily accomplished by treating a solution of Ia, in a non-reactive

solvent such as DMF. with excess trimethylsilyl bromide and stirring at about room temperature for about 4 to 6 hours. Volatiles are removed by concentration in vacuo to a residual material which is treated with water to generate the desired diacid product Ic.

In Synthetic Scheme II, shown below, Q is hydroxy.

Synthetic Scheme II

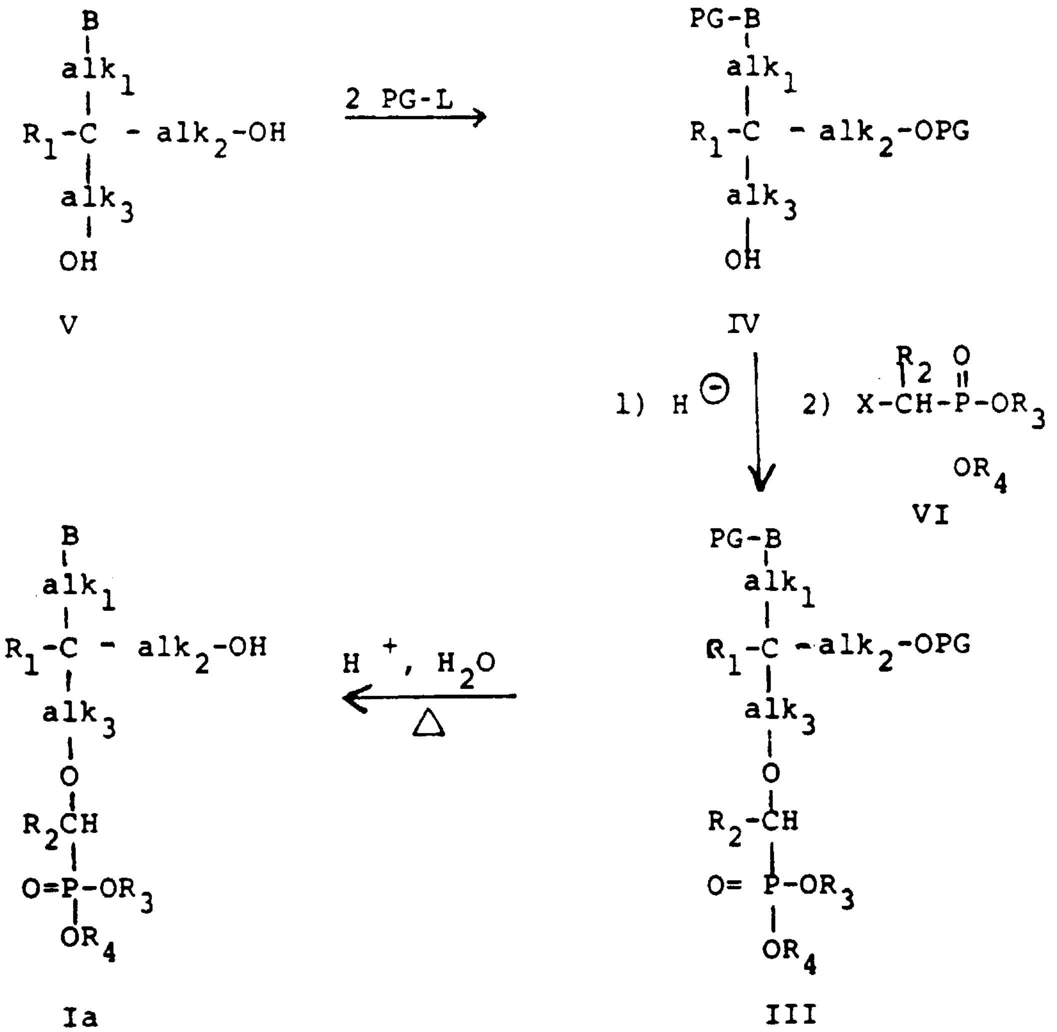


V

IV

VI

Ia

III

It should also be apparent to one skilled in the art that compounds of Formula I wherein Q is hydroxy can also be made in some instances by the Scheme I process. An example of such a synthesis is shown below as Scheme III.

Scheme III

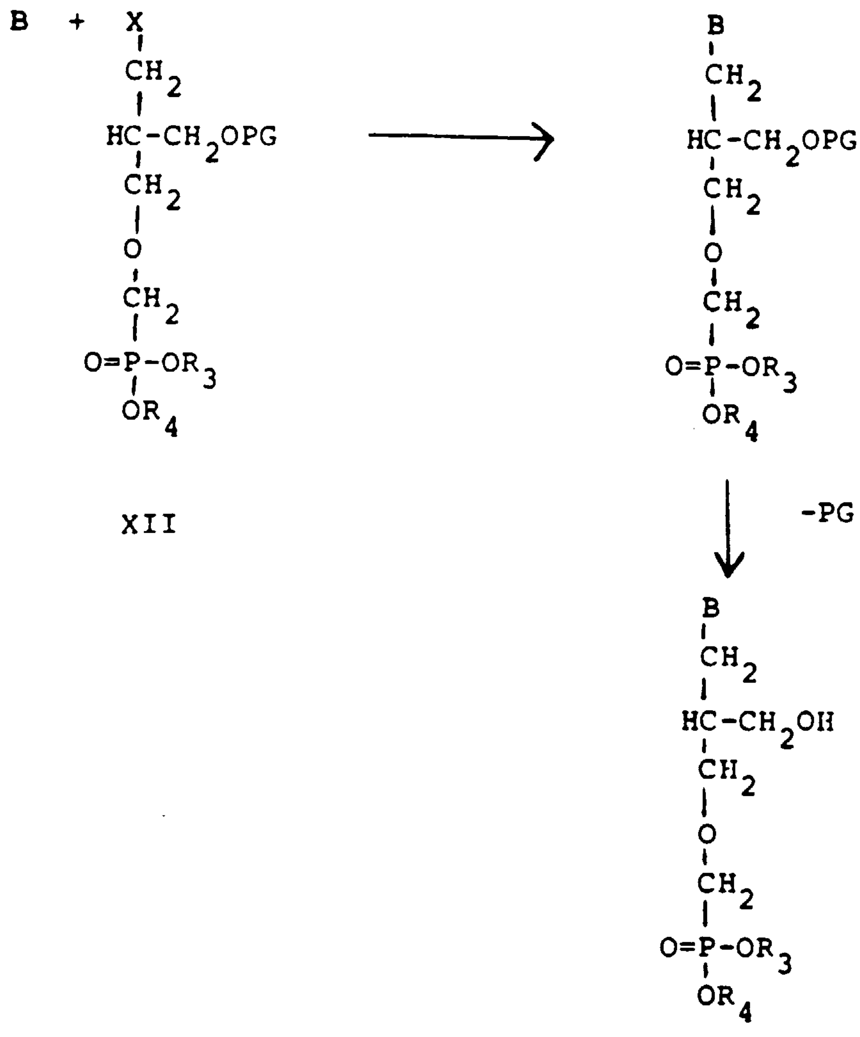


XII

I

An advantage is using the process of Schemes I and III resides in the versatility of using intermediates of Formula II and XII; these may be coupled with a desired base selected from among a large group of such bases to give a variety of Formula I compounds in only one to three steps.

In the foregoing Scheme II B, $alk_1$, $alk_2$, $alk_3$, $R_1$, $R_2$ $R_3$, and $R_4$, are the same as defined hereinabove. The symbol PG represents an organic synthetic protecting group with preferred protecting groups belonging to the triphenylmethyl class of protecting groups. The symbol L is a synthetic organic leaving group which can be selected from the group defined for Synthetic Scheme I with halide preferred and chloride most preferred. In Synthetic Scheme II, $alk_3$ is either a chemical bond or is identical to $alk_2$. Scheme II comprises protecting the amino group moiety of the purine or pyrimidine base or the hydroxy moiety of the pyrimidine base as well as the terminal hydroxy group attached to $alk_2$. In general, this protecting group introduction reaction is carried out in reaction-inert solvents usually containing an excess of a basic reagent such as triethylamine whose function is to scavenge the leaving group anion and hydrogen ion which are liberated as the reaction proceeds. The resulting di-protected intermediate compound of Formula IV is treated with a metal hydride, e.g. NaH, followed by reaction with a phosphonate diester intermediate of Formula VI to give intermediate III. Removal of the protecting groups from intermediate III, done by either heating III in acidic media or by means of mild hydrogenolysis, results in the desired diester product Ia. It should also be obvious to one skilled in the art that this Ia product wherein Q is OH could be converted to a corresponding compound wherein Q = H by conversion of the hydroxy group to a leaving group (as by treatment with tosyl chloride or mesyl chloride) followed by hydride

reduction to a branched alkyl product of Formula Ia wherein $alk_2$ is $C_{1-4}$ alkylene and Q is H.

The reaction intermediates of Formula II, V, and VI which were utilized in Synthetic Schemes I and II, are either commercially available or can be readily synthesized. Representative syntheses of these intermediates are given below in Schemes IV and V.

Scheme IV

Intermediate Compound Synthesis

Intermediate Compounds of Formula II

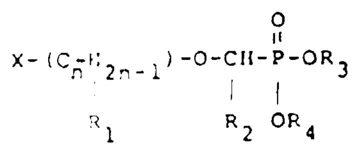

II

wherein

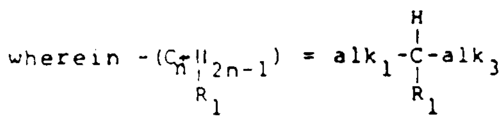

a) n = 2

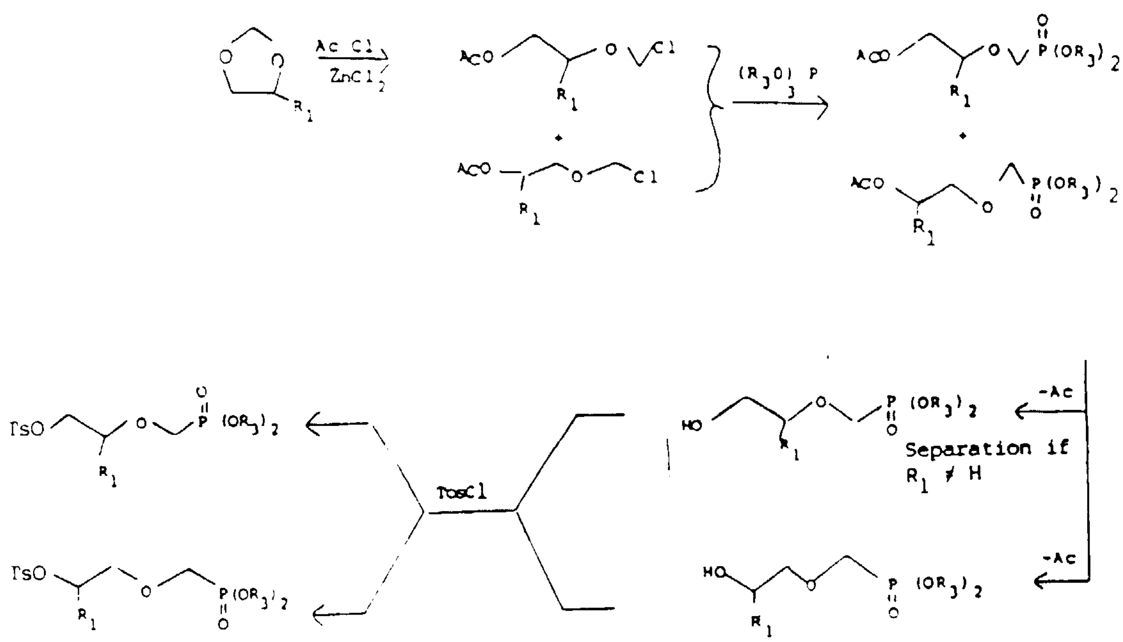

Scheme IV (cont.)

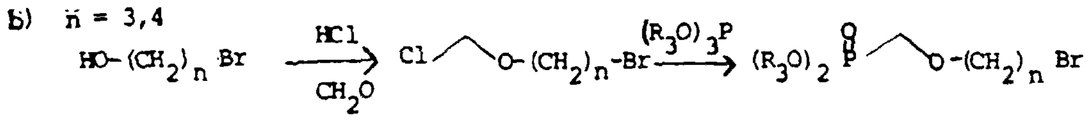


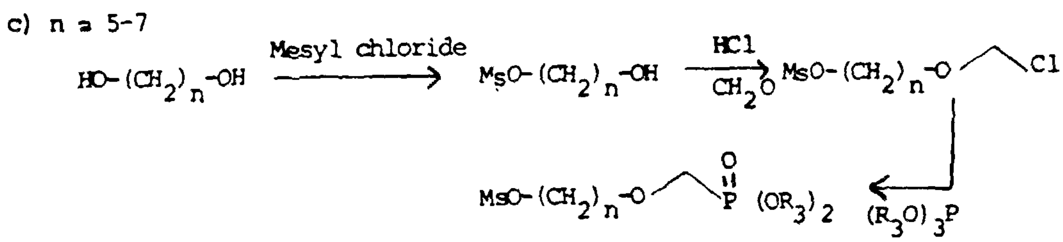


II

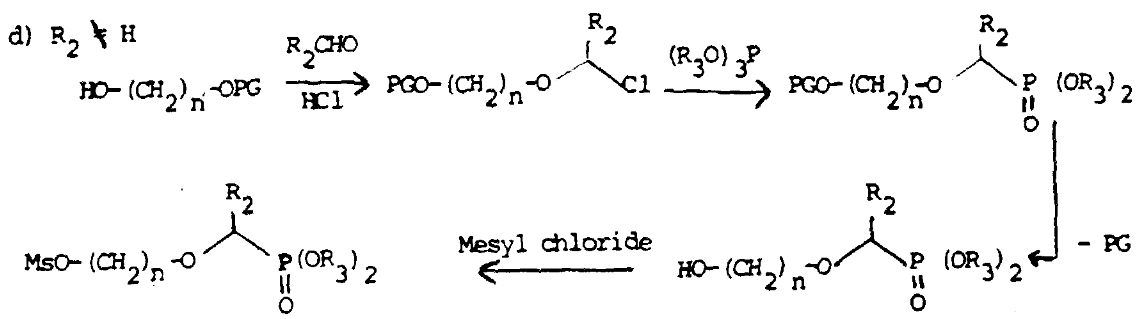


II

## Scheme IV (cont.)

Intermediate Compounds of Formula XII

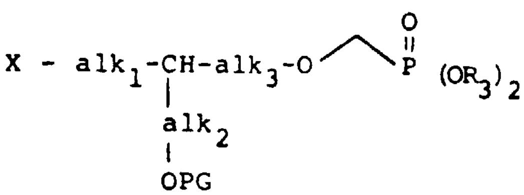

XII

A representative synthesis:

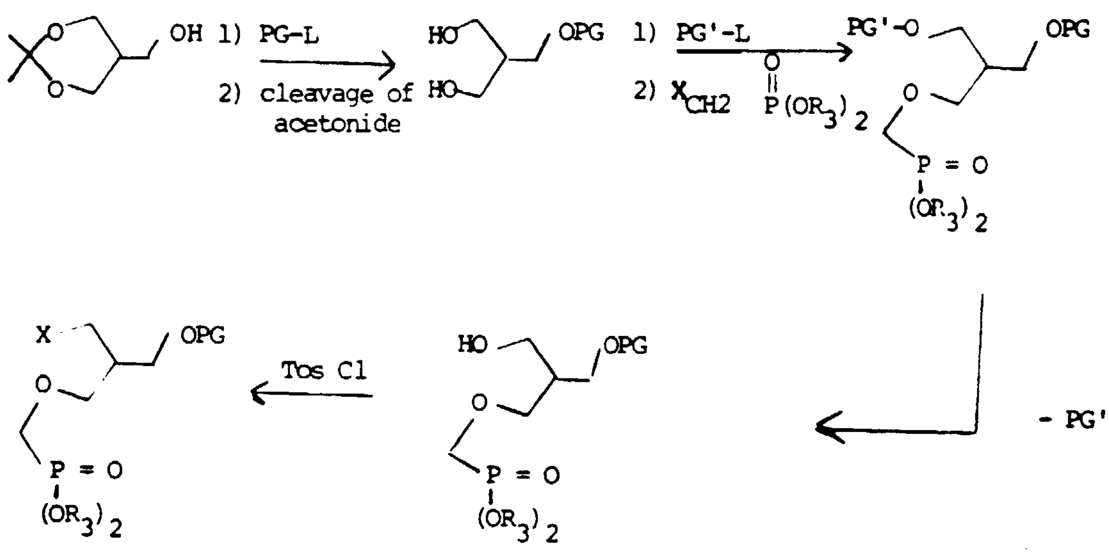


XII

In Scheme IV, n is an integer from 1 to 7 and all other symbols are as previously defined or are conventional, e.g.

$$\mathrm{Ac} = \text{acetyl} = \mathrm{CH_3}\overset{\overset{\mathrm{O}}{\|}}{\mathrm{C}}\text{-},$$

etc. Reactions wherein a terminal hydroxy group is to be converted to a leaving group, e.g. -OH→ -OTos, should be understood to be only representative as other sulfonate leaving group moieties, e.g. mesylate, triflate, can be used in place of tosylate or the -OH functionality can be converted to other types of leaving groups, e.g. halide.

In the example process shown for synthesis of an intermediate compound of formula XII, PG' is a more labile protective group than PG. This allows selective removal of PG' in the presence of PG. Examples of such pairs of protective groups would be: PG' = di-(p-methoxyphenyl) phenylmethyl; PG = triphenylmethyl or PG' = t-butylidimethylsilyl; PG = benzyl.

Scheme V

Intermediate Compound Synthesis

Intermediate Compounds of Formula V

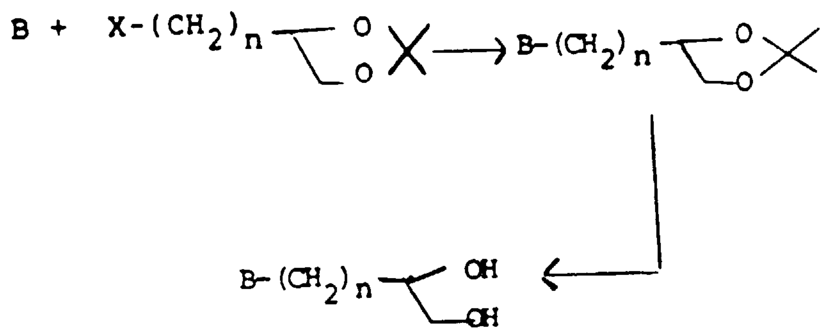

V

The process for preparing Formula V intermediates comprises a first step which is similar to that of Scheme I: generation of the base B anion and alkylation. The resulting alkylenyl acetonide derivative of the base is converted into the target intermediate V by standard acid cleavage of the acetonide moiety.

In summary, the general synthetic processes for preparation of compounds of Formula I comprise:

A.

1) alkylation of a purine or pyrimidine base anion with a leaving group derivative of a diesterified alkylenoxymethylphosphonate intermediate compound (II) to give the corresponding base derivative compound Ia;
2) conversion of Ia to either Ib by acid or base catalyzed hydrolysis or conversion to Ic by treatment of Ia with excess trimethylsilyl bromide, evaporation to dryness and treatment of the residue with water.

B.

1) protection of the reactive ring moiety of the base, e.g. the amino group of adenine or guanine, and a terminal hydroxy group of the starting diol compound V with synthetic organic protecting groups possessing the requisite steric and electronic characteristics appropriate for the necessary selectivity in bonding to give the di-protected intermediate IV;
2) converting the remaining hydroxy group to an oxy anion by treatment of IV with an alkali metal hydride followed by alkylation with a leaving group derivative of a diesterified methylphosphonate intermediate VI thereby giving intermediate III;
3) removal of the protecting groups from intermediate III to provide the phosphonate diester Ia; and 4) same processes as for A.2.).

Physiologically acceptable salts of Formula I compounds of this invention are prepared by methods known in the art. The salts include ammonium salts and salts of physiologically acceptable metals, particularly $Li^+$, $K^+$, $Na^+$, $Ca^{++}$, and $MG^{++}$, and are novel compounds and comprise a further aspect of the invention. Metal salts can be prepared by

reacting the metal hydroxide with a Formula 1 compound of this invention. Examples of metal salts which can be prepared in this way are salts containing $Li^+$, $Na^+$, and $K^+$. A less soluble metal salt can be precipitated from the solution of a more soluble salt by addition of the suitable metal compound. Acid salts may be prepared by reacting a Formula I compound of the invention with an inorganic or organic acid, e.g. HCl, HBr, $H_2SO_4$, and organic sulfonic acids, and the like.

The compounds of this invention, including the physiologically acceptable salts thereof, have desirable antiviral activity. They exhibit activity against DNA viruses, for example, Herpes Simplex virus I, Herpes Simplex virus II, cytomegalovirus, Varicella Zoster virus and also against retroviruses. For use against viral infections, the compounds of this invention can be formulated into pharmaceutical preparations. Such preparations are composed of one or more of the Formula I compounds in association with a pharmaceutically acceptable carrier. The reference Remington's Pharmaceutical Sciences, 15th Edition by E. W. Martin (Mark Publishing Company, 1975) discloses typical carriers and methods of preparation.

The compounds may be administered topically, or systemically. By systemic administration is intended. oral, rectal, and parenteral (i.e. intramuscular, intravenous, subcutaneous and nasal) routes. Generally, it will be found that when a compound of the present invention is administered orally, a larger quantity of the reactive agent is required to produce the same effect as the smaller quantity given parenterally. In accordance with good clinical practice, it is preferred to administer the instant compounds at a concentration level that will produce effective antiviral effects without causing any harmful or untoward side effects. Therapeutically the instant compounds are given as pharmaceutical compositions comprised of an effective antiviral amount of a compound of Formula I or a pharmaceutically acceptable salt thereof and a pharmaceutically acceptable carrier, as stated hereinabove. Pharmaceutical compositions for effecting such treatment will contain a major or minor amount, e.g. from 95 to 0.5% of at least one compound of the present invention in combination with the pharmaceutical carrier, the comprising one or more solid, semisolid, or liquid diluent, filler, and formulation adjuvant which is non-toxic, inert and pharmaceutically acceptable. Such pharmaceutical compositions are preferably in dosage unit forms; i.e. physically discreet units containing a predetermined amount of the drug corresponding to a fraction or multiple of the dose which is calculated to produce the desired therapeutic response. Other therapeutic agents can also be present. Pharmaceutical compositions providing from about 1 to 50 mg. of the active ingredient per unit dose are preferred and are conventionally prepared as tablets, lozenges, capsules, powders, aqueous or oily suspensions, syrups, elixirs, and aqueous solutions. Preferred oral compositions are in the form of tablets or capsules and may contain conventional excipients such as binding agents. (e.g. syrup, acacia, gelatin, sorbitol, tragacanth or polyvinylpyrrolidone), fillers (e.g. lactose, sugar, maize-starch, calcium phosphate, sorbitol, or glycine), lubricants (e.g. magnesium stearate, talc, polyethylene glycol or silica), disintegrants (e.g. starch) and wetting agents (e.g. sodium lauryl sulfate). Solutions or suspensions of a Formula 1 compound with conventional pharmaceutical vehicles are employed for parenteral compositions such as an aqueous solution for intravenous injection or an oily suspension for intramuscular injection. Such compositions having the desired clarity, stability and adaptability for parenteral use are obtained by dissolving from 0.1% to 10% by weight of the active compound in water or a vehicle consisting of a polyhydric aliphatic alcohol such as glycerine, propylene glycol, and polyethylene glycol or mixtures thereof. The polyethylene glycols consist of a mixture of non-volatile, usually liquid, polyethylene glycoles which are soluble in both water and organic liquids and have molecular weights from about 200 to 1500.

Considering the biological activities possessed by the compounds of the instant series, it can be een that these compounds have antiviral properties particularly suited to their use in combating viral infections. Thus, another aspect of the instant invention concerns a process for treating viral infections in a mammal in need of such treatment which comprises systemic or topical administration to such mammal of an effective dose of a Formula I compound or a pharmaceutically acceptable salt thereof. On the basis of testing, an effective dose could be expected to be from about 0.01 to about 30 mg/kg body weight with about 1 to about 20mg/kg body weight a preferred dosage range. It is envisioned that for clinical application compounds of the instant invention will be administered in the same manner as for the reference drug acyclovir. For clinical applications, however, the dosage and dosage regimen must in each case be carefully adjusted, utilizing sound professional judgment and consideration of the age, weight and condition of the recipient, the root of administration and the nature and gravity of the illness. Generally a daily oral dose will comprise from about 150 to about 750 mg, preferably 250 - 500 mg. of a Formula I compound administered from one to three times a day. In some instances, a sufficient therapeutic effect can be obtained at lower doses while in others, larger doses will be required.

Description of the Specific Embodiments

The compounds which constitute this invention and their methods of preparation will appear more fully from a consideration of the following examples which are given for the purpose of illustration only and are not to be construed as limiting the invention in sphere or scope. All temperatures are understood to be in degrees C when not specified. The nuclear magnetic resonance (NMR) spectral characteristics refer to chemical shifts ($\delta$) expressed in parts per

million (ppm) versus tetramethylsilane (TMS) as reference standard. The relative area reported for the various shifts in the proton NMR spectral data corresponds to the number of hydrogen atoms of a particular functional type in the molecule. The nature of the shifts as to multiplicity is reported as broad singlet (bs), singlet (s), multiplet (m), doublet (d), doublet of doublets (dd), triplet (t), or quartet (q). Abbreviations employed are DMSO-$d_6$ (perdeuterodimethylsulfoxide), $CDCl_3$ - (deuterochloroform) and are otherwise conventional. The infrared (IR) spectral descriptions include only absorption wave numbers ($cm^{-1}$) having functional group identification value The IR determinations were employed using potassium bromide (KBr) as diluent. All compounds gave satisfactory elemental analyses.

I. Synthesis of Intermediates

A. Formula V Compounds

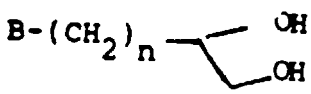

V

Example 1

9-(S)-(2,3-Dihydroxy)propylguanine

A 250 mL 3-necked round bottomed flask fitted with a gas inlet, was oven dried, flushed with argon, and charged with sodium hydride (1.82gm, 0.045mol, 60% by weight in oil). The sodium hydride was washed twice with 50 mL of dry pentane ($CaH_2$), once with dry THF (Na/benzophenone), and covered with dry dimethylformamide (250mL, distilled from $P_2O_5$). 2-Amino-6-benzyloxypurine (10.00 gm, 0.041mol, prepared from 2-aminopurin-6-yl-trimethylammonium chloride) was added in one batch, and the solution heated at 60° for 1 h. Isopropylidene-D-glycerol-γ-tosylate (11.86gm, 0.041 mol, Fluka) was then added in one batch, followed by a catalytic amount (lgm) of sodium iodide, and the resulting mixture heated for 12h at 60°. The solution was then cooled and the volatiles removed under reduced pressure. Thin layer chromatographic analysis of the crude mixture revealed the presence of the N-9 isomer (Rf 0.7 in 10% methanol/methylene chloride) and the N-7 isomer (Rf 0.3 in same). Chromatography over silica gel eluting with ethyl acetate gave 10gm of the N-9 isomer as a gum, and 2gm of the crystalline N-7 isomer, mp. 184-186° (80% overall yield, 5:1 ratio of N-9/N-7).

A solution of(S)-2',3'-O-isopropylidene-6-O-benzyl-9-(2,3-dihydroxy)propylguanine (5.0gm, 0.0139mol) in 80% aqueous acetic acid (80mL) was heated on a steam bath for 1h. The volatiles were then removed in vacuo, and from the residue remaining were evaporated four 100mL volumes of absolute ethanol followed by two 100mL volumes of toluene. The white solid material obtained was recrystallized from water and dried at 5mm for 12h. to yield 2.8gm (89%) of 9-(S)-(2,3-dihydroxy)propylguanine as a white solid, mp. above 260°.
1H NMR (360MHz,DMSO-$d_6$) δ10.57(s,1H), 7.58 (s,1H),6.44(brs, 2H), 5.05(d,J=5Hz, 1H), 4.77(t, J=5Hz, 1H), 4.07(d, J=11Hz, 1H), 3.77 (2 overlapping m, complex 2H), 3.35(m,complex, 1H), 3.27(m,complex, 1H); $^{13}C$ NMR (90MHz, DMSO-$d_6$) 156.90, 153.47, 151.32, 138.36, 116.38, 69.77, 63.51, 46.10; UV (0.1N aq. HCl)λmax 253(ε = 12,305),λmax 272)ε = 8.495); 0.1N aq. NaOH) λmax 245(ε = 10,096),λmax 267 (ε = 10,614; $[\alpha]_D^{25}$ = -45 degrees,

$$[\alpha]_{456}^{25} = -54 \text{ degrees}$$

(c = 0.5,DMSO); IR(KBr) 3180 (br,s), 3100(s), 1695, 1650$cm^{-1}$;
Analysis. Calculated for $C_8H_{11}N_5O_3$: C,42.66; H,4.92; N,31.09. Found: C,42.42; H,4.91; N,30.40.

B. Formula XIII Compounds

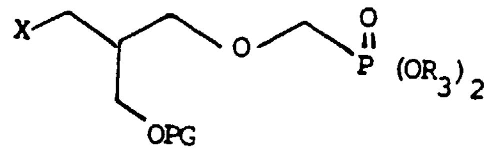

XII

Example 2

2-Benzyloxymethyl-3-diethylphosphonomethoxy-1-(p-toluenesulfonyloxy)propane

A solution of 5-hydroxymethyl-2,2-dimethyl-1,3-dioxane (cf:Bates, H. A.; Farina, J.; Tong, M. J. Org. Chem. 1986, 51, 2637, 3.0 g, 20.5mmol) in 25mL of dry DME under argon was added via cannula to a slurry of NaH (0.740g, 80% dispersion in oil, 24.6mmol) in 60mL of dry DME cooled to 0°C. The resulting grey slurry was stirred at room temperature for 0.5h, then recooled to 0°C, and treated with a solution of benzylbromide (4.56g, 26.7mmol) in 20mL of DME. The reaction mixture was stirred at room temperature (rt) overnight and then quenched with 100ml of $H_2O$. The aqueous layer was separated and extracted with two portions of ethyl acetate. The combined organic layers were then washed with saturated sodium chloride solution, dried over $MgSO_4$, filtered, and concentrated to provide a yellow oil. Purification by column chromatography on silica gel (ethyl acetate/hexane) afforded 3.51g (72%) of 5-benzyloxymethyl-2,2-dimethyl-1,3-dioxane as a clear colorless liquid.

A mixture of 5-benzyloxymethyl-2,2-dimethyl-1,3-dioxane (3.40g, 14.4 mmol) and a few crystals of p-toluenesulfonic acid monohydrate in 100 mL of methanol was stirred at room temperature for 20h. The methanol was removed in vacuo and the residual oil purified by column chromatography on silica gel (ethyl acetate) to give 2.25g (80%) of 2-benzyloxymethyl-1,3-propanediol as a colorless, clear liquid.

NaH (0.87g, 80% dispersion in oil, 29.1 mmol) was washed three times with dry pentane, dried in vacuo, and then suspended in 60mL of dry THF. A solution of 2-benzyloxymethyl-1,3-propanediol (5.70g, 29.Immol) in 5mL of THF was next added dropwise over 20 min. and the reaction mixture stirred at room temperature for 1.5 hrs. to give a white slurry. t-Butyldimethylsilylchloride (4.38g, 29.1 mmol) was then added portionwise over 3 min. and the reaction mixture stirred at room temperature for 2 hours further. The mixture was next diluted with 150mL of ethyl acetate and washed with 10% aqueous potassium carbonate and brine, dried over $MgSO_4$, filtered, and concentrated to give a colorless oil. Purification by column chromatography on silica gel (ethyl acetate/hexanes) provided 7.41 g (82%) of 2-benzyloxymethyl-3-t-butyldimethylsiloxy-1-propanol as a clear, colorless liquid.

A solution of 2-benzyloxymethyl-3-t-butyldimethylsiloxy-1-propanol (5.05g, 16.3mmol) in 10 mL of dry THF was added dropwise over 10 minutes to a slurry of NaH (0.59g, 80% dispersion in oil, 24.4 mmol) in 70mL of dry THF at 0° C under argon. Upon completion of the addition, the ice-bath was removed and the reaction mixture stirred for 45 minutes at room temperature. A solution of diethyl phosphonomethyltrifluoromethane sulfonate (Kluge, A.F. Org. Synthesis 1985 64, 80; Phillion, D.P; Andrew, S.S. Tetrahedron Lett. 1986, 27, 1477; 5.85g, 19.5mmol) in 10mL of dry THF was then added over 5 minutes. After 3 hours at room temperature, the reaction mixture was heated at 50°C for 2 hours and then cooled to room temperature. The reaction was next quenched by addition of 50mL $H_2O$, diluted with $CH_2Cl_2$, and washed with $H_2O$ and saturated sodium chloride solution, dried over $MgSO_4$, filtered, and concentrated. The crude oil was purified by column chromatography on silica gel (EtOH/EtOAc) to provide 2.55g of 2-benzyloxymethyl-1-t-butyldimethylsiloxy-3-(diethylphosphonomethoxy)propane as a colorless oil.

$^1$H NMR indicates that the compound is 80% pure. The major contaminant is unreacted diethyl phosphonomethyl triflate.

Tetrabutylammonium fluoride (8.3mL, 1M in THF, 8.3mmol) was added dropwise to a solution of 2-benzyloxymethyl-1-t-butyldimethylsiloxy-3-(diethylphosphonomethoxy) propane (2.55g, 5.5mmol) in 20mL of THF at room temperature. The reaction mixture was stirred at room temperature for 1.5 hours and then concentrated in vacuo to give 5.6g of a yellow oil. Purification by column chromatography on silica gel (3-5% ethanol in ethyl acetate) provided 1.72g (31% from 2-benzyloxymethyl-3-t-butyldimethylsiloxy-i-propanol) of 2-benzyloxymethyl-3-diethylphosphonomethoxy-1-propanol as a clear colorless oil.

A solution of 2-benzyioxymethyl-3-diethylphosphonomethoxy-1-propanol (0.25g, 0.72mmol) in 5mL of $CH_2Cl_2$ was

cooled to 0°C and treated with triethylamine (0.22g, 2.16mmol). A solution of p-toluenesulfonyl chloride (0.151g, 0.79 mmol) in 2mL of $CH_2Cl_2$ was added next and the reaction mixture allowed to warm gradually to room temperature. After 14 hours at room temperature, the mixture was diluted with $CH_2Cl_2$ and washed with two portions of 10% aqueous HCl and saturated sodium chloride solution, dried over $MgSO_4$, filtered, and concentrated to give an orange oil. Purification by column chromatography on silica gel (1-3% ethanol in ethyl acetate) provided 0.295g of 2-benzyloxymethyl-3-diethylphosphonomethoxy-1-(p-toluenesulfonyloxy)propane as a pale yellow oil.

$^1H$ NMR (200MHz,$CDCl_3$): 7.79(d,J = 8.4Hz, 2H), 7.21-7.39(m,7H),4.41 (brs,2H), 4.06-4.21 (m,6H), 3.71 (d,J = 9Hz, 2H), 3.60(AB quartet, 2H), 3.48 (AB quartet,2H), 2.44 (brs,3H), 2.31 (septet, J = 5.8Hz,1H) and 1.32 (t,J = 7Hz,6H).

$^{13}C$ NMR (50 MHz,$CDCl_3$): 144.7, 137.9, 132.9, 129.8, 127.9, 127.6, 127.4, 73.2, 70.9, and 70.7, 68.3, 67.2, 67.1 and 63.8, 62.5 and 62.3, 39.7, 21.7, and 16.6 and 16.5.

IR(film): 3100, 3080, 3040, 3000, 2920, 2880, 1600, 1500, 1480, 1460, 1395, 1360, 1260, 1200, 1180, 1100,1060, 1040, 980, 840, 820, 800, 750, 710 and 680 $cm^{-1}$.

C. Formula II Compounds

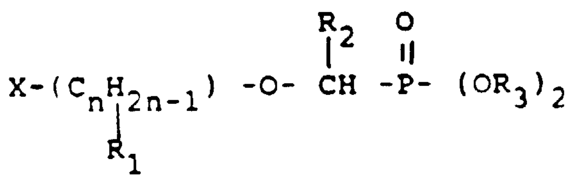

wherein

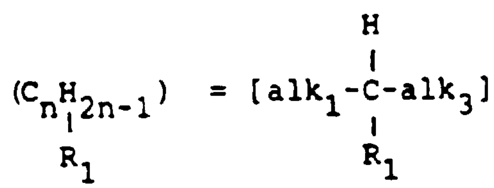

Example 3

<u>1-Methanesulfonyloxy-2-(diethylphosphonomethoxy)ethane</u>

A solution of acetyl chloride (43.2g, 550 mmol) in 100 mL of dry ether was added dropwise over 1 hour to a solution of 1,3 dioxolane (37.1g, 500 mmol) in 300 mL of ether containing a few crystals of zinc (II) chloride at room temperature under nitrogen. The reaction mixture was stirred at room temperature for an additional 2 hours and then concentrated in vacuo. The product was purified by distillation (0.6 mmHg, 56-58°C) to provide 67.9g (89%) of 1-acetoxy-2-(chloromethoxy)ethane as a clear colorless oil. cf: Foye, W.O.; Kaufmann, J.M.; Kim, Y. H. J. Heterocyclic Chem. <u>1982</u>, <u>19</u>, 497.

A mixture of 1-acetoxy-2-(chloromethoxy)ethane (67.8g, 444 mmol) and triethylphosphite (81.3g, 490 mmol) was heated at 105-110°C for 12 hours. Vigorous gas evolution was noted initially. The reaction mixture was next cooled to room temperature and the crude material purified by distillation (0.9mmHg, 130-134°C) to afford 76.9g (68%) of 1-acetoxy-2-(diethylphosphonomethoxy)ethane as a colorless liquid.

15 mL of concentrated hydrochloric acid was added in one portion to a solution of 1-acetoxy-2-(diethylphosphonomethoxy)ethane (76.5g. 300 mmol) in 600 mL of absolute ethanol and the resulting mixture was heated at 55°C for 12 hours. The reaction mixture was then cooled to room temperature and concentrated in vacuo. The resulting clear liquid could be used without purification or purified by distillation (1.5mmHg, 128-132°C) to give 52.1g (82%) of 2-diethylphosphonomethoxy-1-ethanol.

A solution of 2-diethylphosphonomethoxy-1-ethanol (40.7g, 192 mmol) in 500 mL of $CH_2Cl_2$ was cooled to 0°C and then triethylamine (29.1g, 288 mmol) was added in one portion, followed by addition of methanesulfonyl chloride (26.4g, 230 mmol) dropwise over 20 min. The reaction mixture was kept at 0°C for 0.5 hours and then poured into water. The aqueous phase was extracted with two portions of $CH_2Cl_2$ and the combined organic phase dried over $MgSO_4$, filtered and concentrated to afford 54.4g (98%) of 1-methanesulfonyloxy-2-(diethylphosphonomethoxy)ethane

as a clear, pale orange oil. The mesylate could be employed without purification or purified by column chromatography on silica gel (5% methanol in $CH_2Cl_2$).

$^1$H NMR (200MHz, $CDCl_3$): 4.46-4.50 (m,2H), 4.26 (quintet, J=6.8Hz,4H), 3.92-3.99(m,4H), 3.20 (s,3H), and 1.40 (t, J = Hz, 6H).

Example 4

1-Bromo-4-(diethylphosphonomethoxy)butane

To a stirred solution of 49.5g (323 mmol) of 1-bromo-4-butanol and 27.8mL of 37% formaldehyde at 0° is slowly added anhydrous hydrogen chloride gas. The temperature is maintained at -5 to 0° during the slow 6 hour addition. The reaction mixture was then diluted with 500 mL of $Et_2O$ and washed with 2 × 200 mL of ice water. The organic solution was dried ($MgSO_4$) and evaporated. The residue was distilled (55-60° /0.2 mm) to obtain 29g (45%) of 1-bromo-4-(chloromethoxy)butane as a colorless oil.

$^1$H NMR ($CDCl_3$ δ 5.51 (s,2H), 3.71 (t,2H), 3.49(t,2H), 1.98(m,2H), 1.80(m,2H).

To a slurry of 6.26g (149 mmol) of 57% NaH and 300 mL of n-pentane at 0°C was added 17.14g (124 mmol) of diethylphosphite and the mixture was stirred for 1 hour at 0°. The mixture was then cooled to -70 and 25g (124 mmol) of 1-bromo-4-(chloromethoxy)butane was added and the reaction mixture warmed to 0° and stirred for 1 hour. The mixture was then filtered and evaporated. The residue was purified by $SiO_2$ chromatography to give 26g (70%) of 1-bromo-4-(diethylphosphonomethoxy)butane as a colorless oil.

$^1$H NMR ($CDCl_3$) δ 4.18 (m,4H), δ 3.77 (d,2H), 3.61 (t,2H), 3.45 (t,2H), 1.95 (m,2H), 1.79 (m,2H), 1.35 (t,6H).

Example 5

1-(Diethylphosphonomethoxy)-5-(methanesulfonyloxy)pentane

To a solution of 85.0g (0.904 mole) of 1,5-pentanediol and 30.3g (0.30 mole) of triethylamine in 350 mL of dry $CH_2Cl_2$ at -20°C was added dropwise a solution of 28.5g (0.25 mole) of methanesulfonylchloride in 100 mL of $CH_2Cl_2$ over 2 hours under nitrogen atmosphere. The solution was stirred at 2 hours at -20°C and then at -4°C for 18 hours. the reaction mixture was washed with $H_2O$, 1N HCl, $H_2O$, then dried and evaporated. The residual oil was chromatographed on a silica gel column, eluting with EtOAc-$CH_2Cl_2$ (2:8)-After combining the appropriate fractions there was obtained 25.7g (56.5%) of 5-hydroxypentylmethylsulfonate as a colorless oil.

$^1$H NMR ($CDCl_3$) 4.25 (t,J = 6.2Hz,2H), 3.65 (t, J = 5.4Hz,2H), 3.03 (s,3H), 2.35 (s,1H), and 1.75-1.85 (m,6H).

A mixture of 5-hydroxypentylmethylsulfonate (18.2 g 0.1 mole) and trioxane (3.6g, 0.036 mole) in dichloroethane (30mL) was saturated with dry HCl over a period of 2.5 hours with cooling (-10°C). The resulting mixture was dried ($MgSO_4$) and filtered and the solvent evaporated in vacuo. A white oil (24g) was obtained which could not be distilled in vacuo because of decomposition but was reacted as unpurified chloromethoxy intermediate.

$^1$H NMR ($CDCl_3$) 5.51 (s, 2H,), 4.28 (t, J = 5Hz, 2H), 3.68 (t, J = 5.8 Hz,2H) 3.02 (s, 3H,) and 1.40 to 1.80 (m, 6H)

Sodium hydride (6.16gh, 0.154 mole as a 50% oil dispersion prewashed with n-pentane) was slurried in 100 mL n-pentane. The solution was cooled to 0°C and a solution of 20.34g (0.147 mole) diethylphosphite in 10mL n-pentane was added dropwise over 20 mm. The slurry was cooled to -78°C. To this cold slurry was added a solution of the unpurified 5-chloromethoxy-1-methanesulfonoxypentane (31.0g, 0.134 mole) in 120mL THF with vigorous stirring. After the addition was completed the mixture was warmed to -15° in 2 to 3 hours. It was diluted with 500 mL ethyl acetate, washed with $H_2O$, dried over $MgSO_4$ and evaporated to dryness. The resulting oil was chromatographed through a silica gel column (10% EtOAc-$CH_2Cl_2$) to yield 22.5g of a colorless oil (47%).

$^1$H NMR ($CDCl_3$) 4.3 (m, 6H), 3.8 (d, 2H), 3.6 (t, 2H), 3.0 (s, 3H), and 1.4-1.8 (m, 12H).

II. Synthesis of Products

Example 6

9-(2-(Diethylphosphonomethoxy)ethyl)guanine(Ia)

A mixture of $N^2$-acetyl guanine (6.47g, 33.5mmol), 2-(diethylphosphonomethoxy)-1-iodoethane (9.80g, 30.4mmol) and potassium carbonate (8.41g, 60.9mmol) in 350mL of dry DMF was heated at 100°C for 4h. The reaction mixture was then allowed to cool at rt and any insoluble material was removed by filtration. The filtrate was concentrated in vacuo to give a viscous yellow oil which was purified by column chromatography on silica gel (5-10% methanol in $CH_2Cl_2$). Recrystallization of combined fractions containing the desired product from ethyl acetate afforded a total of

1.50g (13%) of 2-N-acetyl-9-(2'-(diethylphosphonomethoxy) ethylguanine as a white crystalline solid, m.p. 140.5-141.5°C.

Analysis: Calculated for $C_{14}H_{22}N_5O_6P$•1/2$H_2O$: C, 42.42%, H, 5.85%; N, 17.67%. Found: C, 42.33%; H, 5.60%; N, 17.99%.

2-N-Acetyl-9-(2 -(diethylphosphonomethoxy)ethyl)guanine (1.42g, 3.68mmol) was dissolved in 50mL of 40% aqueous methylamine and the solution was stirred at rt for 45 min. The reaction mixture was concentrated in vacuo and evaporated three times with toluene to give a gummy, white solid. The crude material was stirred in hot ethyl acetate for 1h, then cooled to rt, and the product collected by filtration to provide 1.19g of 9-(2 -(diethylphosphonomethoxy) ethyl)guanine

$^1$H NMR (200MHz,$d_6$-DMSO): 10.4-10.7(brs, 1H), 7.65(s, 1H), 6.46 (brs, 2H), 4.14(t, J=7Hz, 2H), 3.99-(quintet, J = 6Hz, 4H), 3.78-3.89 (m, 4H), and 1.20 (t, J = 7Hz, 6H).

$^{13}$CNMR(50.3MHz,$d_6$-DMSO): 156.7, 153.4, 151.1, 137.5, 116.3, 70.5 and 70.2, 65.4 and 62.2, 61.7 and 61.6, 42.1, and 16.2 and 16.1.

IR(KBr): 3200 (br), 3160, 3000, 1700, 1620, 1545, 1480, 1380, 1255, 1180, 1110, 1060, 1030, 900, 820, and 795 $cm^{-1}$.

Analysis. Calculated for $C_{12}H_{20}N_5O_5P$•1/2$H_2O$: C, 40.68%; H, 5.98%; N, 19.77% Found: C, 40.61%; H, 5.74%; N, 19.79%.

Example 7

9-(2-(Monoethylphosphonomethoxy)ethyl)guanine(Ib)

9-(2-(diethylphosphonomethoxy)ethyl)guanine (0.198g, 0.57mmol) was dissolved in 15 mL of 1N sodium hydroxide solution and the mixture was stirred at room temperature for 1 hour. The solution was then acidified with 10% aqueous hydrochloric acid to pH 1 and concentrated in vacuo. Residual salts were removed by reverse phase column chromatography (C18 adsorbent, elution with water) to provide 0.150g of 9-(2 -(ethylphosphonomethoxy)ethyl)guanine as a white crystalline solid, mp = 192.5 - 193.5°C.

$^1$H NMR (200 MHz, $d_6$-DMSO): 10.6 (brs, 1H), 7.69 (s,1H), 6.48 (brs, 2H), 4.12 (t, J = 5.2Hz,2H), 3.89(quintet, j = 7.2Hz,2H), 3.81 (t, J = 5.2Hz,2H), 3.68 (d, J = 8.6Hz, 2H), and 1.15 (t, J = 7.2Hz, 3H).

Example 8

8-Bromo-9-(2'-(phosphonomethoxy)ethyl)guanine(Ic)

Bromine (1mL) was added to 100mL of water and the mixture stirred vigorously at rt until all the bromine had dissolved (15 min.). 9-(2'-(diethylphosphonomethoxy)ethyl)guanine (0.360g, 1.04mmol) was then dissolved in 10ml $H_2O$ and treated dropwise with the aqueous bromine solution until the color of $Br_2$ persisted. The reaction mixture was allowed to stand at 0°C for 1h and then was concentrated to afford a dark yellow viscous gum. Purification was accomplished by column chromatography on silica gel (MeOH-$CH_2Cl_2$) to provide 0.31g of 8-bromo-9-(2'-(diethylphosphonomethoxy)ethyl)guanine as an orange powder.

$^1$H NMR (200MHz, $d_6$-DMSO): 6.58(brs, 2H), 4.12(t, J=5Hz, 2H), 3.95(quintet,J = 7Hz,4H), 3.74-3.85(m, 4H), and 1.17 (t, J = 7Hz, 6H).

$^{13}$C NMR (50.3MHz, $d_6$-DMSO): 155.4, 153.7, 152.4, 120.9, 116.6, 69.7 and 69.5, 65.7 and 62.5, 61.8 and 61.6, 43.1, and 16.2 and 16.1.

Bromotrimethylsilane (0.47g, 3.1mmol) was added dropwise over 5 min. to a solution of 8-bromo-9-(2'-(phosphonomethoxy)ethylguanine) (0.13g, 0.31mmol) in 3mL of DMF at rt under argon in a foil-covered flask. The reaction mixture was stirred at rt for 4h and then the solvent and excess silane were removed in vacuo. The resulting orange oil was treated with $H_2O$ and acetone to provide a fine pale yellow solid which was collected by filtration. The solid was purified by recrystallization from H20/EtOH to give 8-bromo-9-(2'-(phosphonomethoxy)ethyl) guanine as 21 mg of pale yellow crystals.

$^1$H NMR (200MHz, $d_6$-DMSO): 10.6(brs, 1H), 6.63(brs, 2H), 4.10(t, J=5Hz, 2H), 3.79(t, J=5Hz, 2H), and 3.57(d, J = 8Hz, 2H).

$^{13}$C NMR (50.3MHz, $d_6$-DMSO): 155.4, 153.8, 152.4, 120.8, 116.7, 69.3 and 69.2, 68.2 and 65.0, and 42.9.

Example 9

9-(3(Monoethylphosphonomethoxy)propyl)guanine (Ib)

A solution of 9-(3-diethylphosphonomethoxy)-6-O-(methoxyethyl)guanine (Ia 417 mg,lmmol) in 10 mL of 3N HCl

was heated at 85° for 3.5 hours. The solvent was removed using high vacuum to give 400 mg of the glassy monoester product.
$^1H$ NMR ($D_2O$) 7.95 (s, 1H), 4.38 (t, 2H), 4.15 (quintet, 2H), 3.85 (d,2H), 3.70 (t, 2H), 2.25 (m, 2H), and 1.30 (t, 3H).

Example 10

9(4-(phosphonomethoxy)butyl)adenine (IC)

To a slurry of 0.962g (22.8mmol) of 57% NaH in 150 ml of distilled DMF was added in one portion 3.363 gm (24.9 mmol) of adenine. The mixture was heated at 80° for 1 hr. and then cooled to 30° and 6.30g (20.7mmol) of 4-(diethylphosphonomethoxy)-1-bromobutane was added and the mixture was warmed to 60° and stirred for 2 hrs. The solvent was then removed under high vacuum and the residue was triturated three times with 100 ml of $CH_2Cl_2$ and filtered. The combined filtrates were evaporated and purified by SiO2 chromatography to give 4.9 g (66%) of Ia product as a white crystalline material mp 67°.
$^1H$ NMR ($CDCl_3$) 6 8.25(s, 1H), 7.80 (s,H), 6.50 (s,2H), 4.10 (m,6H), 3.67 (d,2H), 3.52 (t,2H), 1.91 (m,2H), 1.53 (m, 2H), 1.23 (t,6H).
UV $\lambda$ max (MeOX) 261 mm ($\varepsilon$ 14155)
Analysis: Calculated for $C_{14}H_{24}N_5O_4P$: C, 47.02; H, 6.77; N, 19.60. Found: C, 46.81; H, 6.83; N, 19.69.

To a solution of 3.3g (9.2 mmol) of the Ia product in 75 mL of distilled DMF was added 13mL (90mmol) of bromotrimethylsilane. The solution was stirred at 20° for 5 hrs. and then concentrated in vacuo. The residue was crystallized from 30mL $H_2O$ to give 2.5g (90%) of Ic product as a white crystalline material, mp 238° C.
$^1H$ NMR ($D_2O$) $\delta$ 8.02 (s, 1H), 8.00 (s, 1H), 4.13 (t,2H), 3.66 (m,4H), 1.84 (m,2H), 1.67 (m,2H)
UV $\lambda$ max (MeOH) 261 ($\varepsilon$ 13824)
Analysis: Calculated for $C_{10}H_{14}N_5O_4P$: C,39.87; H, 5.35; N, 23.25. Found: C,39.46; H,5.08; N,23.17.

Example 11

9-(4-(phosphonomethoxy)butylguanine (Ic)

To a slurry of 560 mg (70mmol) of LiH in 200 ml of distilled DMF was added 8.0g (41mmol) of 6-O-(methoxyethyl) guanine.(Kjellberg, J.; Liljenberg, M.; Johannson, N.G. Tetrahedron Lett. 1986, 27, 877.) The mixture was stirred at 20°C for 1.5 hrs. and then 12.6g (41.5mmol) of 4-(diethylphosphonomethoxy)-1-bromobutane in 5 ml of DMF was added and the mixture was heated at 60°C for 4.5 hrs. The reaction mixture was then cooled to 10°C and treated dropwise with dilute HCl to pH8. The solvents were then removed under high vacuum and the crude residue was purified by $SiO_2$ chromatography to give 4.2 g of the 0-6-protected Ia guanine product as a light yellow oil.
$^1H$ NMR ($CDCl_3$) $\delta$ 7.65 (s,1H), 4.92 (s,2H), 4.66 (t,2H), 4.15 (m,6H), 3.81 (m,4H), 3.62 (t,2H), 3.45 (s,3H), 1.96 (m, 2H), 1.62 (m,2H), 1.35 (t,6H).

A solution of 3.0gm (7.Ommol) of Ia intermediate 6-0-(methoxyethyl)-9-(4-diethylphosphonomethoxy)-butyl)-guanine in 30 mL of 6N HCl was refluxed for 5.5 hrs. The solvent was then removed under high vacuum and the glassy residue was dissolved in 3mL of $H_2O$ and diluted with acetone until cloudy. After stirring overnight 1.6 gm (73%) of crystalline Ic product was obtained, mp 240°.
$^1H$ NMR ($D_2O$) $\delta$ 7.813 (s,1H), 4.07 (t, 2H), 3.59 (m, 4H), 1.89 (m, 2H), 1.61 (m, 2H).
UV max ($H_2O$) 271 $\varepsilon$ = 8494
Analysis: Calculated for $C_{10}H_{16}N_5O_5P$: C, 37.85; H, 5.08; N,22.07. Found: C,38.26; H,5.00; N,21.45.

Example 12

1-(4-(phosphonomethoxy)butyl)thymine (Ia)

To a slurry of 0.634g (15 mmol) 57% NaH in 80 ml of distilled DMF was added in one portion 2.07 g-(16.4mmol) of thymine. The mixture was heated at 80° for 1 hour. The reaction mixture was then cooled to 60°C and to it was added 4.15g (13.7 mmol) of 4-diethylphosphonomethoxy)-1-bromobutane and the mixture was warmed to 90° for 1 hour. The solvent was then removed under high vacuum, the residue was triturated with 3 x 100 mL $CH_2Cl_2$ and the fractions were combined and filtered. The residue was purified by $SiO_2$ chromatography to give 2.2 g (46%) of IA product as a colorless oil.
$^1H$ NMR ($CDCl_3$) $\delta$ 7.01 (s,1H), 4.07 (m,4H) 3.52 (t,2H), 1.82 (s,3H) 1.69 (m,2H) 1.55 (m,2H), 1.25 (t,6H).

To a solution of 2.0g (5.75 mmol) of the diethyl phosphonate (IA) in 50 mL of distilled DMF was added 7.6 mL of bromotrimethylsilane. The solution win stirred 16 hours at 20°C and the solvents wore then removed under high vacuum. The glassy residue was crystallized from $H_2O$-acetone to give 810 my (48%) of white crystalline IC product mp 140°.

$^1H$ NMR ($D_3O$) δ 7.46 (s,1H), 3.73 (t,2H), 3.64 (d,2H) 3.58 (t,2H), 1.82 (s,3H), 1.69 (m,2H), 1.56 (m,2H).

By utilization of the foregoing examples which can be appropriately modified to produce the intermediate or product structure sought, such modifications being obvious to one skilled in the art; other examples of compounds embraced by the present invention can be prepared. As can be seen, the monoester compounds Ib and diacid compounds Ic are obtained readily from the diester precursors la. Additional examples of Formula Ib and Ic compounds which are prepared by the methods disclosed herein are shown in Tables 1, 2 and 3. The corresponding la analogs are intended to be understood as well.

**TABLE 1**

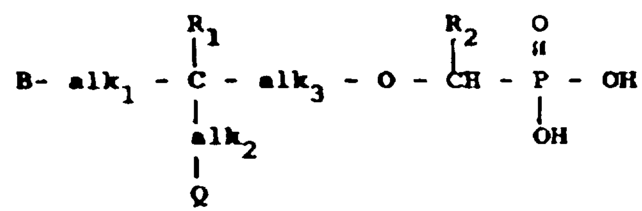

Ic

| EX | B[a)] | Alk$_1$[b)] | Alk$_2$ | Alk$_3$ | Q | $R_1$ | $R_2$ | MP (°C) |
|---|---|---|---|---|---|---|---|---|
| 13 | G | - | - | $CH_2$ | H | Me | H | 210 (dec) |
| 15 | G | $C_2H_4$ | $CH_2$ | - | OH | H | H | |
| 16 | G | $CH_2$ | $CH_2$ | $CH_2$ | OH | H | H | 246.5-247.5 |
| 17 | G | - | - | - | H | H | H | |
| 18 | G | $CH_2$ | - | $CH_2$ | H | H | H | 280-285 |
| 19 | G | $C_2H_4$ | - | $CH_2$ | H | H | H | 240 (dec) |
| 20 | G | $C_2H_4$ | - | $C_2H_4$ | H | H | H | 174-177 |
| 21 | G | $C_3H_6$ | - | $C_2H_4$ | H | H | H | 240 (dec) |

Table 1 (Continued)

| EX | B | $Alk_1$ | $Alk_2$ | $Alk_3$ | Q | $R_1$ | $R_2$ | MP (°C) |
|---|---|---|---|---|---|---|---|---|
| 22 | G | $C_4H_8$ | - | $C_2H_4$ | H | H | H | 228 (dec) |
| 23 | G | $CH_2$ | - | - | H | H | Me | >260 |
| 24 | G | $CH_2$ | - | - | H | Me | H | |
| 25 | 2-Aminopurine | $CH_2$ | - | - | H | H | H | 258-260 |
| 26 | G | $CH_2$ | $CH_2$ | - | H | H | H | |

a) G = guanine, T = thymine, C = cytosine, U = uracil.
b) - = a chemical bond.

**TABLE 2**

**Additional Compounds of Formula Ic**

| EX | B | $Alk_1$ | $Alk_2$ | $Alk_3$ | Q | $R_1$ | $R_2$ |
|---|---|---|---|---|---|---|---|
| 27 | 8-$NH_2$G | $CH_2$ | - | - | H | H | H |
| 28 | 8-MeG | $CH_2$ | - | - | H | H | H |
| 29 | T | $CH_2$ | - | - | H | Me | H |
| 30 | C | $CH_2$ | - | $CH_2$ | H | H | Me |
| 31 | U | $CH_2$ | - | - | H | H | Me |

G = guanine, T = thymine, C = cytosine, U = uracil.

**TABLE 3**

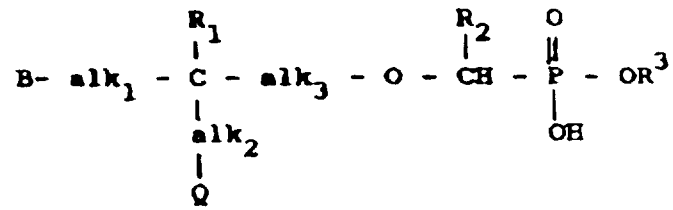

Ib

| EX | B | Alk1 | Alk2 | Alk3 | Q | R1 | R2 | R3 | MP (°C) |
|---|---|---|---|---|---|---|---|---|---|
| 32 | G | $CH_2$ | - | - | H | H | H | $CH_3$ | 199 (dec) |
| 33 | G | $CH_2$ | - | - | H | H | H | $CH_2CH_2CH_3$ | 195-197 |
| 34 | G | $CH_2$ | - | - | H | H | H | $CH(CH_3)_2$ | 222.5-224 |
| 35 | G | $CH_2$ | - | - | H | H | Me | $CH_2CH_3$ | |
| 36 | 2-Aminopurine | $CH_2$ | - | - | H | H | H | $CH_2CH_3$ | |
| 37 | G | $CH_2$ | $CH_2$ | - | OH | H | H | $CH_2CH_3$ | |
| 38 | G | $CH_2$ | $CH_2$ | - | H | H | H | $CH_2CH_3$ | |
| 39 | C | $CH_2$ | $CH_2$ | - | OH | H | H | $CH_2CH_3$ | |
| 40 | G | - | - | $CH_2$ | H | Me | H | $CH_2CH_3$ | 80 (dec) |

G = guanine, C = cytosine

III. Biological Testing

Example 42

Testing and evaluation of compounds against herpes virus.

A. Plaque Reduction Assay

Herpes simplex virus (HSV) strains wars grown and titered at 37°C in vero cells (African Green Monkey Kidney cells) and used for virus work before the tenth passage.

Cells were grown and maintained in Earle's Minimum Essential Medium (EMEM), Gibco Laboratories, supplemented with 0.75% sodium bicarbonate, 2mM 1-glutamine, Pen-strep. and 5-10% fetal calf serum.

The titer of HSV strains is determined by a plaque titration method (Roizman and Roane,"Virology," 15:75-79, 1961). Tissue culture 24-well petri dishes are seeded with cells and used for assays when approximately 75% monolayer. Volumes (0.1ml) of logarithmic dilutions of the virus strain are inoculated onto each of triplicate wells, and absorbed for one hour with intermittent shaking. The inoculum thereafter is removed, and 1 ml of 5-10% EMEM containing 0.3% human immune serum globulin is added. After a 48 hr. incubation period at 37°C in a 5% $CO_2$ atmosphere, the overlay medium is removed and the cell sheets stained with Giemsa stain. The number of plaques is counted, the triplicate is averaged, and the number of plaque-forming units per ml is calculated.

The compounds are tested for activity against the herpes simplex strains using a stock solution of each compound freshly prepared. Appropriate dilution of each compound are made in 10% EMEM before usage. The antiviral efficacy of each compound is determined using the plaque reduction assay described above. Briefly, tissue culture 24-well plates, with approximately 75% cell monolayer are inoculated with approximately 50 plaque forming units of HSV per 0.1 ml, and the virus adsorbed for 1 hr, with intermittent shaking. After removal of the inoculum, 1 ml of 10% EMEM containing two-fold dilutions of the appropriate drug are added in triplicates. Triplicate wells/plate receives no drug and are used as a virus control. After a 48-hour incubation period, at 37°C in a 5% $CO_2$ atmosphere, the overlay medium is removed, the cells stained as described above, and plaques are counted. The counts of triplicate wells are averaged, and the number of plaques in the presence of each drug dilution are calculated.

The antiviral potency of the drug is determined by $ID_{50}$, the drug concentration necessary to reduce the number of plaques by 50% of those in the virus control cultures.

B. Colorimetric Dye-Uptake Assay

For the primary screening, a colorimetric dye-uptake assay (McLaren, C., et al., "Antiviral Research," 3:323, 1983) is used employing rapidly growing vero cells. Briefly, cells, compound and virus dilutions are added onto 96-well tissue culture plates simultaneously using the cells, viruses and medium described above. After 48 hr. incubation at 37°C in 5% $CO_2$ atmosphere, the overlay medium is removed and the cell sheets are stained with 0.04% neutral red solution. After 30 min. incubation at 37°C, the cell sheets are washed and the stain is eluted with 0.05 M sodium monophosphate in 47% ethanol and the O.D. is determined at 540 nm wave length.

The 50% inhibitory dose (ID50) for each drug is determined by linear regression analysis.

Example 43

Testing and evaluating of compounds against human cytomegalovirus.

Human cytomegalovirus (HCMV) strain (AD169) was grown and titered at 37° in human embryonic lung (diploid) cells, MRC-5, and used for the antiviral assay.

The compounds are tested for activity against the HCMV using the procedure for the plaque reduction assay described above.

Example 44

Testing and evaluating of compounds against Murine Retroviruses:

The compounds wore evaluated for antiviral activity against Murine leukemia virus (MuLV) strains using the UV-XC plaque assay (Rowe, et al., "Virology," 42:1136, 1970).

The MuLV strains were grown in feral mouse cells (SC-1) and used for antiviral tests using the UV-XC plaque assay. Briefly, SC-1 coils are grown as monolayers in 4-well tissue culture plates and inoculated with approximately

50-100 plaque forming units of MuLV in 0.5 ml of 5% EMEM containing 20 ug/ml DEAE/Dextran. After 1 hr. adsorbtion, the inoculum is removed and 5 ml of 5% EMEM containing three-fold dilutions of the appropriate drug are added. Five days later, the cultures are UV-irradiated with an ultraviolet lamp and rat XC sarcoma cells are added to the cultures. Three-four days after UV-irradiation, the cell cultures are stained with Giemsa stain and the plaques are enumerated. Antiviral activity is expressed in terms of the reduction in the mean number of UV-XC plaques counted in the drug treated, virus-infected cultures compared with mean number of plaques counted in untreated, virus-infected control cultures. The $ID_{50}$ for each drug was determined.

Some representative antiviral test data are displayed in Table 4.

TABLE 4

Antiviral Test Results of Some Representative Formula I Compounds*

| | Dye-Uptake | | Plaque Reduction | | CMV | Muly |
|---|---|---|---|---|---|---|
| Reference Cmpds, | HSV-1 | HSV-2 | HSV-1 | HSV-2 | | |
| Acyclovir | 0.5 | 1.0 | 0.3 | 1.5 | | N.T. |
| 9-(1,3-Dihydroxypropoxymethyl) guanine | | | | | 1.2 | |
| 3'-Azido-3'-deoxythymidine | N.T. | N.T. | N.T. | N.T. | | 0.0001 |
| (S)-9-(3-Hydroxy-2-phosphonomethoxy)propyl adenine | 15.1 | > 25 | 13.8 | 35.8 | 0.13 | 2.0 |
| Formula I Compounds | | | | | | |
| Ex. 7 | 12 | 8 | 4.8 | 5.1 | 0.23 | 0.56 |
| Ex. 13 | 8.7 | 10.4 | 3.0 | 3.3 | | |
| Ex. 18 | | | | | 6.0 | 0.04 |
| Ex. 25 | | | | | <0.032 | |
| Ex. 32 | 1.5 | 3.8 | 1.6 | 1.2 | | 0.05 |
| Ex. 33 | | | 9.7 | 9.9 | | |
| Ex. 34 | | | 74.9 | 34.8 | | 1.72 |

* All results expressed as $ID_{50}$.

## Claims

**Claims for the following Contracting States : AT, BE, CH, DE, FR, GB, IT, LI, LU, NL, SE**

**1.** A compound of formula I

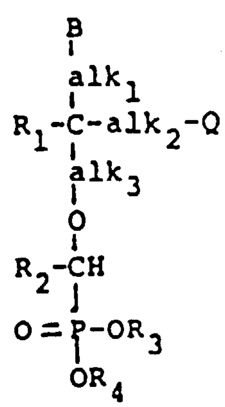

I

wherein B is a purine or pyrimidine base selected from the group consisting of xanthine, hypoxanthine, guanine, 8-bromoguanine, 8-chloroguanine, 8-aminoguanine, 8-hydrazinoguanine, 8-hydroxyguanine, 8-methylguanine, 8-thioguanine, 2-aminopurine, 2,6-diaminopurine, cytosine, 5-ethylcytosine, 5-methylcytosine, thymine, uracil, 5-bromouracil, 5-ioduracil, 5-ethyluracil, 5-propyluracil, 5-vinyluracil, and 5-bromovinyluracil:

> $alk_1$, $alk_2$ and $alk_3$ are independently selected from a chemical bond or $C_1$-$C_4$ alkylene;
> Q is hydrogen or hydroxyl;
> $R_1$ and $R_2$ are independently selected from hydrogen and $C_1$-$C_4$ alkyl; and $R_3$ and $R_4$ are independently selected from hydrogen, $C_1$-$C_6$ alkyl, phenyl and phenyl-$C_1$-$C_4$ alkylene;
> and the corresponding salts, zwitterions, and/or solvates except those compounds wherein $alk_1$ is methylene, $R_1$ is hydrogen, $alk_2$ is a chemical bond and Q is hydrogen, $alk_3$ is a chemical bond, $R_2$, $R_3$ and $R_4$ are hydrogen and B is uracil-1-yl, thymine-1-yl, cytosine-1-yl, guanine-9-yl, or hypoxanthine-9-yl, and
> except those compounds wherein $alk_1$ is methylene, $R_1$ is hydrogen, $alk_2$ is methylene and Q is hydroxyl, $alk_3$ is a chemical bond, $R_2$, $R_3$ and $R_4$ are hydrogen, and B is uracil-1-yl, cytosine-1-yl, thymine-1-yl, guanine-9-yl, guanine-7-yl, hypoxanthine-9-yl, or 2-aminopurine-9-yl, and except (RS) compounds wherein $alk_1$ and $alk_2$ are methylene, $alk_3$ is a chemical bond, Q is hydroxyl, $R_1$, $R_2$, $R_3$ and $R_4$ are hydrogen and B is 2,6-diaminopurine-9-yl, and except compounds having the formula:

$$B\text{-}CH_2CH_2OCH_2P(O)(OC_2H_5)_2,$$

> wherein B is xanthine, hypoxanthine, guanine, 2-amino-purine-9-yl, 2,6-diaminopurine-9-yl, cytosine, thymine or uracil.

**2.** A compound of Claim 1 wherein B is a purine base, $R_1$, $R_2$ and Q are hydrogen, $alk_1$ and $alk_2$ are methylene and $alk_3$ is a chemical bond.

**3.** A compound of Claim 2 wherein the purine base is a guanine moiety.

**4.** A compound of Claim 1 wherein B is a pyrimidine base.

**5.** A compound of Claim 2 wherein $R_1$, $R_2$ and Q are hydrogen

**6.** A compound of Claim 1 wherein $R_3$ and $R_4$ are hydrogen.

**7.** A compound of Claim 1 wherein one of $R_3$ and $R_4$ is hydrogen and the other is $C_1$-$C_6$ alkyl.

**8.** The compounds of Claim 1, namely

> 9-(3-(phosphonomethoxy)propyl)-guanine;
> 9-(4-(phosphonomethoxy)butyl)-guanine;
> 8-bromo-9-(2-(phosphono-methoxy)ethyl)guanine;
> 1-(4-(phosphonomethoxy)-butyl)thymine;
> 9-(1-methyl-2-(phosphonomethoxy)-ethyl)guanine;
> 9-(2-(phosphonomethoxy)-1-propyl)guanine;
> 9-(2-hydroxymethyl-3-(phosphonomethoxy)propyl)guanine;
> 8-methyl-9-(2-(phosphonomethoxy)-ethyl)guanine;
> 9-(4-hydroxy-3-(phosphonomethoxy)butyl)guanine;
> 9-(2-(monoethylphosphonomethoxy)-ethyl)guanine;
> 9-(2-(monomethylphosphonomethoxy)-ethyl)guanine;
> 9-(2-(mono-n-propylphosphonomethoxy)ethyl)guanine;
> 9-(2-(mono-isopropylphosphonomethoxy)ethyl)guanine;
> 9-(2-(1-phosphono-1-ethoxy)ethyl)guanine;

**9.** A pharmaceutical composition for antiviral use comprising an effective antiviral amount of at least one compound of Claims 1 to 8 in admixture with a pharmaceutically acceptable carrier.

**10.** A process for preparing the compounds of Claim 1 to 8 which comprises

A) for the preparation of the compounds of formula I wherein

Q is hydrogen and $alk_2$ is a chemical bond converting a purine or pyrimidine base B which is as defined in Claim 1 to an anion by treatment with a base,
reacting the so obtained anion of the purine base B with a phosphonate diester of the general formula II:

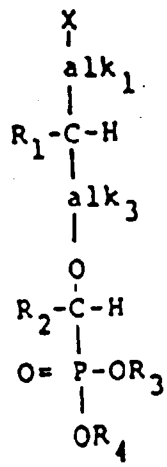

II

wherein X is a standard organic leaving group;
$alk_1$, $alk_3$, $R_1$ and $R_2$ are as defined in Claim 1;
$R_3$ and $R_4$ are as defined in Claim 1 except being hydrogen, to obtain a phosphonate diester of the general formula Ia

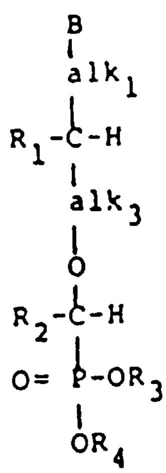

Ia

or

B) for the preparation of the compounds of formula I wherein

Q is hydroxy

a) reacting a compound of the general formula V

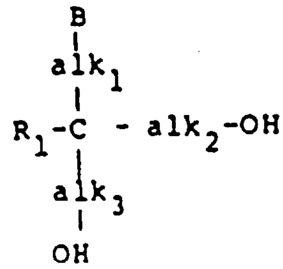

V

with a compound of the general formula PG-L, wherein PG represents an organic protecting group and L is an organic leaving group to obtain a compound of the general formula IV :

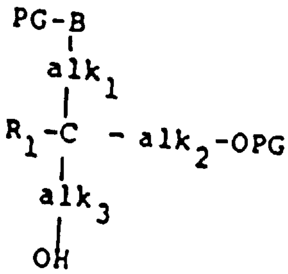

IV

treating the so obtained compound of formula IV with a metal hydride, followed by reaction with a phosphonate diester of formula VI

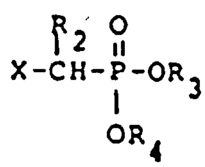

VI

to obtain a compound of the general formula III:

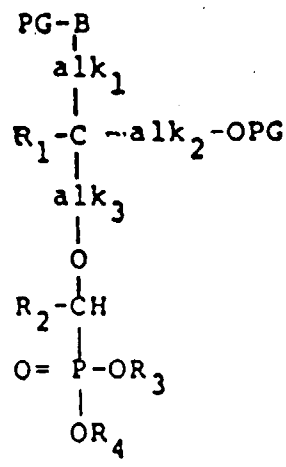

III

removing the protecting groups from the so obtained compound of formula III to obtain a compound of the general formula Ia as defined above, or

b) reacting a purine or pyrimidine base B with a compound of the general formula XII

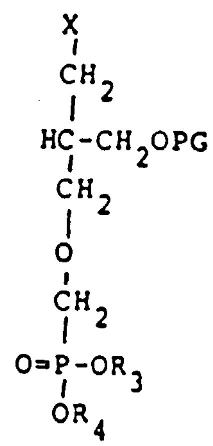

XII

to obtain a compound of the general formula:

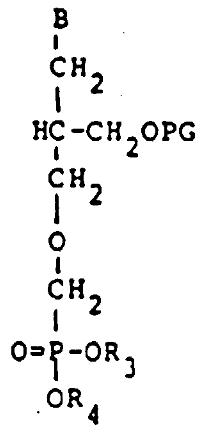

removing the protecting group to obtain a compound of the general formula I'

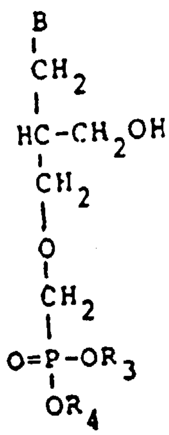

I'

and, if desired, converting the so obtained diester compounds of formlae Ia and I' to the corresponding monoester or diacid compounds.

whereby in the above formulae X, B, $alk_1$, $alk_2$, $alk_3$, $R_1$, $R_2$, $R_3$, $R_4$ and PG are as defined above.

**11.** A process for preparing the pharmaceutical composition of claim 9 which comprises incorporating an effective amount of at least one compound of claims 1 to 8 into a pharmaceutically acceptable carrier.

**Claims for the following Contracting State : ES**

**1.** A process for preparing a compound of formula I

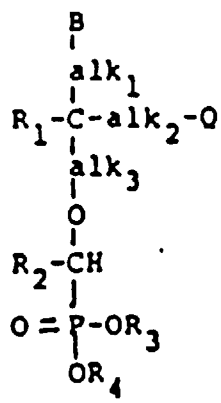

I

wherein B is a purine or pyrimidine base selected from the group consisting of xanthine, hypoxanthine, guanine, 8-bromoguanine, 8-chloroguanine, 8-aminoguanine, 8-hydrazinoguanine, 8-hydroxyguanine, 8-methylguanine, 8-thioguanine, 2-aminopurine, 2,6-diaminopurine, cytosine, 5-ethylcytosine, 5-methylcytosine, thymine, uracil, 5-bromouracil, 5-ioduracil, 5-ethyluracil, 5-propyluracil, 5-vinyluracil, and 5-bromovinyluracil:

$alk_1$, $alk_2$ and $alk_3$ are independently selected from a chemical bond or $C_1$-$C_4$ alkylene;
Q is hydrogen or hydroxyl;
$R_1$ and $R_2$ are independently selected from hydrogen and $C_1$-$C_4$ alkyl; and $R_3$ and $R_4$ are independently selected from hydrogen, $C_1$-$C_6$ alkyl, phenyl and phenyl-$C_1$-$C_4$ alkylene;
and the corresponding salts, zwitterions, and/or solvates except those compounds wherein $alk_1$ is methylene, $R_1$ is hydrogen, $alk_2$ is a chemical bond and Q is hydrogen, $alk_3$ is a chemical bond, $R_2$, $R_3$ and $R_4$ are hydrogen and B is uracil-1-yl, thymine-1-yl, cytosine-1-yl, guanine-9-yl, or hypoxanthine-9-yl, and
except those compounds wherein $alk_1$ is methylene, $R_1$ is hydrogen, $alk_2$ is methylene and Q is hydroxyl, $alk_3$ is a chemical bond, $R_2$, $R_3$ and $R_4$ are hydrogen, and B is uracil-1-yl, cytosine-1-yl, thymine-1-yl, guanine-9-yl, guanine-7-yl, hypoxanthine-9-yl, or 2-aminopurine-9-yl, and except (RS) compounds wherein $alk_1$ and $alk_2$ are methylene, $alk_3$ is a chemical bond, Q is hydroxyl, $R_1$, $R_2$, $R_3$ and $R_4$ are hydrogen and B is 2,6-diaminopurine-9-yl,
and except compounds having the formula:

$$B\text{-}CH_2CH_2OCH_2P(O)(OC_2H_5)_2,$$

wherein B is xanthine, hypoxanthine, guanine, 2-amino-purine-9-yl, 2,6-diaminopurine-9-yl, cytosine, thymine or uracil,

which comprises

A) for the preparation of the compounds of formula I wherein

Q is hydrogen and $alk_2$ is a chemical bond converting a purine or pyrimidine base B which is as defined above to an anion by treatment with a base,
reacting the so obtained anion of the purine base B with a phosphonate diester of the general formula II:

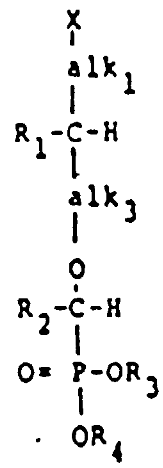

II

wherein X is a standard organic leaving group;
$alk_1$, $alk_3$, $R_1$ and $R_2$ are as defined above ;
$R_3$ and $R_4$ are as defined above except being hydrogen, to obtain a phosphonate diester of the general formula Ia

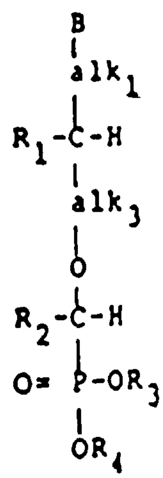

(Ia)

or

B) for the preparation of the compounds of formula I wherein

Q is hydroxy

a) reacting a compound of the general formula V

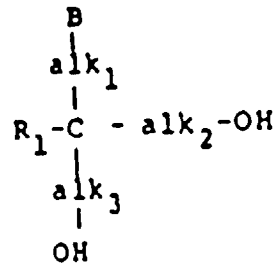

V

with a compound of the general formula PG-L, wherein PG represents an organic protecting group and L is an organic leaving group to obtain a compound of the general formula IV:

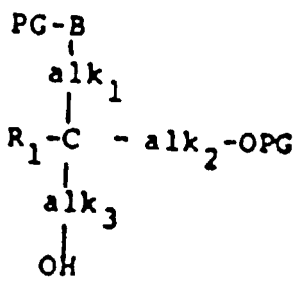

IV

treating the so obtained compound of formula IV with a metal hydride, followed by reaction with a phosphonate diester of formula VI

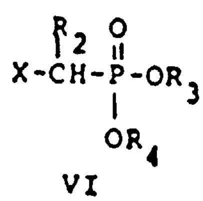

VI

to obtain a compound of the general formula III:

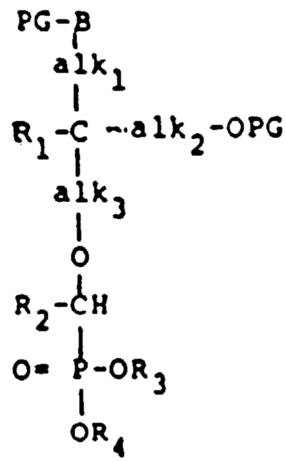

III

removing the protecting groups from the so obtained compound of formula III to obtain a compound of the general formula Ia as defined above, or

b) reacting a purine or pyrimidine base B with a compound or the general formula XII

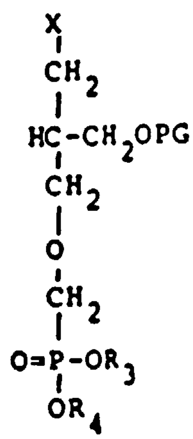

(XII)

to obtain a compound of the general formula:

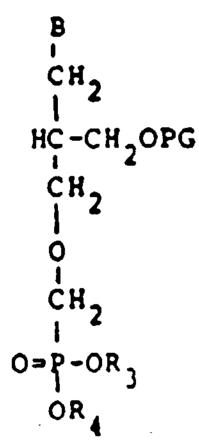

removing the protecting group to obtain a compound of the general formula I'

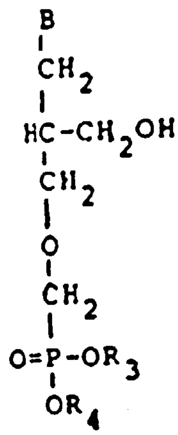

I'

and, if desired, converting the so obtained diester compounds of formlae Ia and I' to the corresponding monoester or diacid compounds.
whereby in the above formulae X, B, $alk_1$, $alk_2$, $alk_3$, $R_1$, $R_2$, $R_3$, $R_4$ and PG are as defined above.

**2.** A process of Claim 1 wherein B is a purine base, $R_1$, $R_2$ and Q are hydrogen, $alk_1$ and $alk_2$ are methylene and $alk_3$ is a chemical bond.

**3.** A process according to Claim 2 wherein the purine base is a guanine moiety.

**4.** A process according to Claim 1 wherein B is a pyrimidine base.

**5.** A process according to Claim 2 wherein $R_1$, $R_2$ and Q are hydrogen.

**6.** A process according to Claim 1 wherein $R_3$ and $R_4$ are hydrogen.

**7.** A process according to Claim 1 wherein one of $R_3$ and $R_4$ is hydrogen and the other is $C_1$-$C_6$-alkyl.

**8.** A process according to Claim 1 for preparing the following compounds:

9-(3-(phosphonomethoxy)propyl)-guanine;
9-(4-(phosphonomethoxy)butyl)guanine;
8-bromo-9-(2-(phosphono-methoxy)ethyllguanine;
1-(4-(phosphonomethoxy)-butyl)thymine;
9-(1-methyl-2-(phosphonomethoxy)-ethyl)guanine;
9-(2-(hosphonomethoxy)-1-propyl)guanine;
9(2-hydroxymethyl-3-(phosphoromethoxy)propyl)guanine;
8-methyl-9-(2-(phosphonomethoxy)-ethyl)guanine;
9-(4-hydroxy-3-(phosphonomethoxy)butyl)guanine;
9(2-(monoethylphosphonomethoxy)-ethyl)guanine;
9-(2-(monomethylphosphonomethoxy)-ethyl)guanine;
9-(2-(mono-n-propylphosphonomethoxy)ethyl)guanine;
9-(2-(mono-isopropylphosphonomethoxy)ethyl)guanine;
9-(2-(1-phosphono-1-ethoxy)ethyl)guanine;

**9.** A process for preparing a pharmaceutical composition which comprises incorporating an effective amount of at least one compound as defined in claims 1 to 8 into a pharmaceutically acceptable carrier.

**Claims for the following Contracting State : GR**

**1.** A compound of formula I

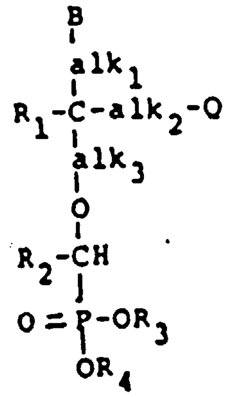

I

wherein B is a purine or pyrimidine base selected from the group consisting of xanthine, hypoxanthine, guanine, 8-bromoguanine, 8-chloroguanine, 8-aminoguanine, 8-hydrazinoguanine, 8-hydroxyguanine, 8-methylguanine, 8-thioguanine, 2-aminopurine, 2,6-diaminopurine, cytosine, 5-ethylcytosine, 5-methtylcytosine, thymine, uracil, 5-bromouracil, 5-ioduracil, 5-ethyluracil, 5-propyluracil, 5-vinyluracil, and 5-bromovinyluracil:

$alk_1$, $alk_2$ and $alk_3$ are independently selected from a chemical bond or $C_1$-$C_4$ alkylene;
Q is hydrogen or hydroxyl;
$R_1$ and $R_2$ are independently selected from hydrogen and $C_1$-$C_4$ alkyl;
and $R_3$ and $R_4$ are independently selected from hydrogen, $C_1$-$C_6$ alkyl, phenyl and phenyl-$C_1$-$C_4$ alkylene;
and the corresponding salts, zwitterions, and/or solvates except those compounds wherein $alk_1$ is methylene, $R_1$ is hydrogen, $alk_2$ is a chemical bond and Q is hydrogen, $alk_3$ is a chemical bond, $R_2$, $R_3$ and $R_4$ are hydrogen and B is uracil-1-yl, thymine-1-yl, cytosine-1-yl, guanine-9-yl, or hypoxanthine-9-yl, and
except those compounds wherein $alk_1$ is methylene, $R_1$ is hydrogen, $alk_2$ is methylene and Q is hydroxyl, $alk_3$ is a chemical bond, $R_2$, $R_3$ and $R_4$ are hydrogen, and B is uracil-1-yl, cytosine-1-yl, thymine-1-yl, guanine-9-yl, guanine-7-yl, hypoxanthine-9-yl, or 2-aminopurine-9-yl, and except (RS) compounds wherein $alk_1$ and $alk_2$ are methylene, $alk_3$ is a chemical bond, Q is hydroxyl, $R_1$, $R_2$, $R_3$ and $R_4$ are hydrogen and B is 2,6-diaminopurine-9-yl, and except compounds having the formula:

$$B\text{-}CH_2CH_2OCH_2P(O)\,(OC_2H_5)_2,$$

wherein B is xanthine, hypoxanthine, guanine, 2-amino-purine-9-yl, 2,6-diaminopurine-9-yl, cytosine, thymine or uracil.

**2.** A compound of Claim 1 wherein B is a purine base, $R_1$, $R_2$ and Q are hydrogen, $alk_1$ and $alk_2$ are methylene and $alk_3$ is a chemical bond.

**3.** A compound of Claim 2 wherein the purine base is a guanine moiety.

**4.** A compound of Claim 1 wherein B is a pyrimidine base.

**5.** A compound of Claim 2 wherein $R_1$, $R_2$ and Q are hydrogen.

**6.** A compound of Claim 1 wherein $R_3$ and $R_4$ are hydrogen.

**7.** A compound of Claim 1 wherein one of $R_3$ and $R_4$ is hydrogen and the other is $C_1$-$C_6$ alkyl.

**8.** The compounds of Claim 1, namely

9-(3-(phosphonomethoxy)propyl)-guanine;
9-(4-(phosphonomethoxy)butyl)guanine;
8-bromo-9-(2-(phosphono-methoxy)ethyl)guanine;
1-(4-(phosphonomethoxy)-butyl)thymine;
9-(1-methyl-2-(phosphonomethoxy)-ethyl)guanine;
9-(2-(phosphonomethoxy)-1-propyl)guanine;
9-(2-hydroxymethyl-3-(phosphonomethoxy)propyl)guanine;
8-methyl-9-(2-(phosphonomethoxy)-ethyl)guanine;
9-(4-hydroxy-3-(phosphonomethoxy)butyl)guanine;
9-(2-(monoethylphosphonomethoxy)-ethyl)guanine;
9-(2-(monomethylphosphonomethoxy)-ethyl)guanine:
9-(2-(mono-n-propylphosphonomethoxy)ethyl)guanine;
9-(2-(mono-isopropylphosphonomethoxy)ethyl)guanine;
9-(2-(1-phosphono-1-ethoxy)ethyl)guanine;

**9.** A process for preparing a pharmaceutical composition which comprises incorporating an effective amount of at least one compound as defined in claims 1 to 8 into a pharmaceutically acceptable carrier.

**10.** A process for preparing the compounds of Claim 1 to 8 which comprises

A) for the preparation of the compounds of formula I wherein

Q is hydrogen and $alk_2$ is a chemical bond converting a purine or pyrimidine base B which is as defined in Claim 1 to an anion by treatment with a base,
reacting the so obtained anion of the purine base B with a phosphonate diester of the general formula II:

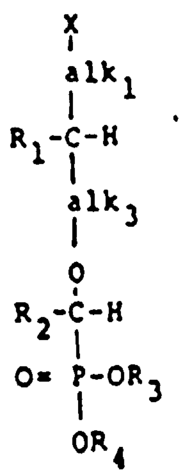

(II)

wherein X is a standard organic leaving group;
$alk_1$, $alk_3$, $R_1$ and $R_2$ are as defined in Claim 1;
$R_3$ and $R_4$ are as defined in Claim 1 except being hydrogen, to obtain a phosphonate diester of the general formula Ia

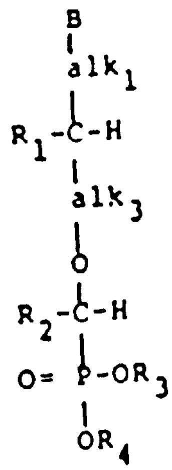

Ia

or

B) for the preparation of the compounds of formula I wherein

Q is hydroxy

a) reacting a compound of the general formula V

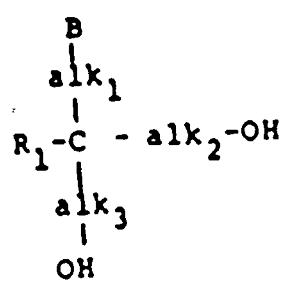

V

with a compound of the general formula PG-L, wherein PG represents an organic protecting group and L is an organic leaving group to obtain a compound of the general formula IV:

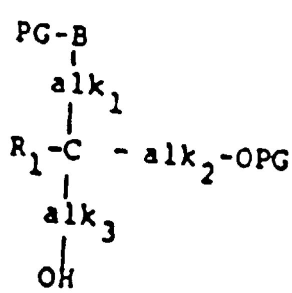

IV

treating the so obtained compound of formula IV with a metal hydride, followed by reaction with a phosphonate diester of formula VI

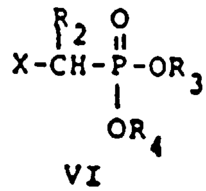

VI

to obtain a compound of the general formula III:

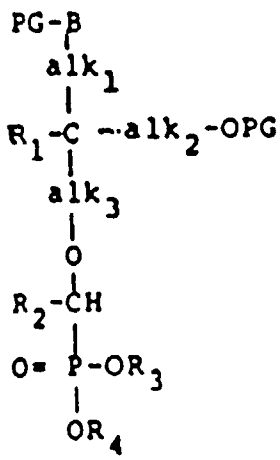

III

removing the protecting groups from the so obtained compound of formula III to obtain a compound of the general formula Ia as defined above, or

b) reacting a purine or pyrimidine base B with a compound of the general formula XII

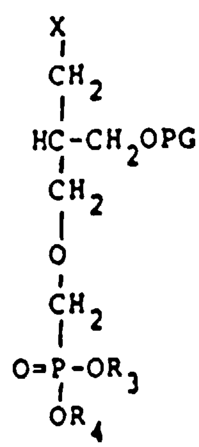

XII

to obtain a compound of the general formula:

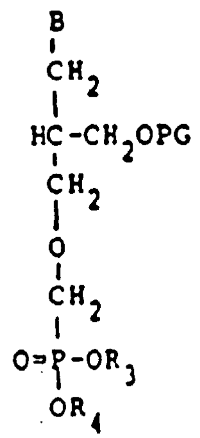

removing the protecting group to obtain a compound of the general formula I'

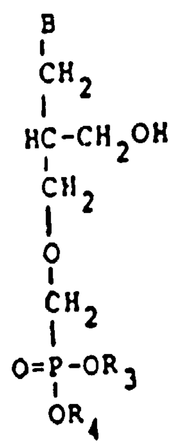

I'

and, if desired, converting the so obtained diester compounds of formlae Ia and I' to the corresponding monoester or diacid compounds.
whereby in the above formulae X, B, $alk_1$, $alk_2$, $alk_3$, $R_1$, $R_2$, $R_3$, $R_4$ and PG are as defined above.

**Patentansprüche**

**Patentansprüche für folgende Vertragsstaaten : AT, BE, CH, LI, DE, FR, GB, IT, LU, NL, SE**

**1.** Verbindungen der Formel I

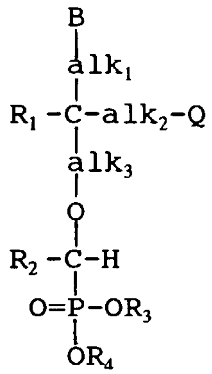

I

worin B für eine Purin- oder Pyrimidinbase, ausgewählt unter Xanthin, Hypoxanthin, Guanin, 8-Bromguanin, 8-Chlorguanin, 8-Aminoguanin, 8-Hydrazinoguanin, 8-Hydroxyguanin, 8-Methylguanin, 8-Thioguanin, 2-Aminopurin, 2,6-Diaminopurin, Cytosin, 5-Ethylcytosin, 5-Methylcytosin, Thymin, Uracil, 5-Bromuracil, 5-Joduracil, 5-Ethyluracil, 5-Propyluracil, 5-Vinyluracil und 5-Bromvinyl-uracil, steht;
$alk_1$, $alk_2$ und $alk_3$ unabhängig voneinander ausgewählt sind unter einer chemischen Bindung oder $C_1$-$C_4$-Alkylen;
Q für ein Wasserstoffatom und Hydroxyl steht;
$R_1$ und $R_2$ unabhängig voneinander ausgewählt sind unter einem Wasserstoffatom und $C_1$-$C_4$-Alkyl; und
$R_3$ und $R_4$ unabhängig voneinander ausgewählt sind unter einem Wasserstoffatom, $C_1$-$C_6$-Alkyl, Phenyl und Phenyl-$C_1$-$C_4$-alkylen;
und die entsprechenden Salze, Zwitterionen und/oder Solvate, ausgenommen diejenigen Verbindungen, worin $alk_1$ für Methylen, $R_1$ für ein Wasserstoffatom, $alk_2$ für eine chemische Bindung und Q für ein Wasserstoffatom stehen, $alk_3$ eine chemische Bindung ist, $R_2$ , $R_3$ und $R_4$ für ein Wasserstoffatom stehen und B für Uracil-1-yl, Thymin-1-yl, Cytosin-1-yl, Guanin-9-yl oder Hypoxanthin-9-yl steht,
und ausgenommen diejenigen Verbindungen, worin $alk_1$ für Methylen, $R_1$ für ein Wasserstoffatom, $alk_2$ für Methylen und Q für Hydroxyl steht, $alk_3$ eine chemische Bindung ist, $R_2$, $R_3$ und $R_4$ für ein Wasserstoffatom stehen und B für Uracil-1-yl, Cytosin-1-yl, Thymin-1-yl, Guanin-9-yl, Guanin-7-yl, Hypoxanthin-9-yl oder 2-Aminopurin-9-yl steht,
und ausgenommen (RS)-Verbindungen, worin $alk_1$ und $alk_2$ für Methylen stehen, $alk_3$ eine chemische Bindung ist, Q für Hydroxyl steht, $R_1$, $R_2$, $R_3$ und $R_4$ für ein Wasserstoffatom stehen und B für 2,6-Diaminopurin-9-yl steht,
und ausgenommen Verbindungen der Formel:

$$B\text{-}CH_2CH_2OCH_2P(O)\,(OC_2H_5)_2,$$

worin B für Xanthin, Hypoxanthin, Guanin, 2-Aminopurin-9-yl, 2,6-Diaminopurin-9-yl, Cytosin, Thymin oder Uracil steht.

2. Verbindungen nach Anspruch 1, worin B für eine Purinbase steht, $R_1$, $R_2$ und Q für ein Wasserstoffatom stehen, $alk_1$ und $alk_2$ für Methylen stehen und $alk_3$ eine chemische Bindung ist.

3. Verbindungen nach Anspruch 2, worin die Purinbase ein Guaninrest ist.

4. Verbindungen nach Anspruch 1, worin B für eine Pyrimidinbase steht.

5. Verbindungen nach Anspruch 2, worin $R_1$, $R_2$ und Q für ein Wasserstoffatom stehen.

6. Verbindungen nach Anspruch 1, worin $R_3$ und $R_4$ für ein Wasserstoffatom stehen.

7. Verbindungen nach Anspruch 1, worin einer der Reste $R_3$ und $R_4$ für ein Wasserstoffatom steht und der andere für $C_1$-$C_6$-Alkyl steht.

8. Verbindungen nach Anspruch 1, nämlich

9-(3-(Phosphonomethoxy) propyl)-guanin;
9-(4-(Phosphonomethoxy)butyl)-guanin;
8-Brom-9-(2-(phosphonomethoxy)ethyl)guanin;
1-(4-(phosphonomethoxy)-butyl)thymin;
9-(1-Methyl-2-(phosphonomethoxy)-ethyl)guanin;
9-(2-(Phosphonomethoxy)-1-propyl)guanin;
9-(2-Hydroxymethyl-3-(phosphonomethoxy)propyl)guanin;
8-Methyl-9-(2-(phosphonomethoxy)-ethyl)guanin;
9-(4-Hydroxy-3-(phosphonomethoxy)butyl)guanin;
9-(2-(Monoethylphosphonomethoxy)-ethyl)guanin;
9-(2-(Monomethylphosphonomethoxy)-ethyl)guanin;
9-(2-(Mono-n-propylphosphonomethoxy)ethyl)guanin;
9-(2-(Mono-isopropylphosphonomethoxy)ethyl)guanin;
9-(2-(1-Phosphono-1-ethoxy)ethyl)guanin .

**9.** Pharmazeutisches Mittel zur antiviralen Anwendung, das eine antiviral wirksame Menge mindestens einer Verbindung nach den Ansprüchen 1 bis 8 zusammen mit einem pharmazeutisch akzeptablen Träger enthält.

**10.** Verfahren zur Herstellung der Verbindungen nach Anspruch 1 bis 8, wobei man

A) zur Herstellung der Verbindungen der Formel I, worin

Q für ein Wasserstoffatom steht und $alk_2$ eine chemische Bindung ist, eine Purin- oder Pyrimidinbase B, die wie in Anspruch 1 definiert sind, durch Behandlung mit einer Base in ein Anion umwandelt;
das so erhaltene Anion der Purinbase B mit einem Phosphonatdiester der allgemeinen Formel II:

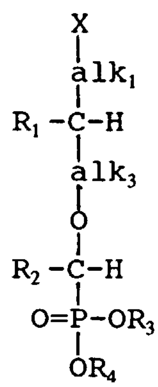

II

worin X eine organische Standardabgangsgruppe ist, $alk_1$, $alk_3$,
$R_1$ und $R_2$ wie in Anspruch 1 definiert sind;
$R_3$ und $R_4$ wie in Anspruch 1 definiert sind, aber kein Wasserstoffatom bedeuten, umsetzt, wobei man einen Phosphonatdiester der allgemeinen Formel Ia

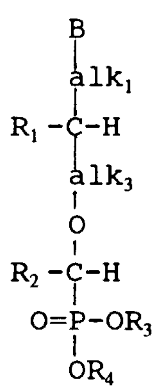

Ia

erhält, oder

B) zur Herstellung der Verbindungen der Formel I, worin

Q für Hydroxy steht,

a) eine Verbindung der allgemeinen Formel V

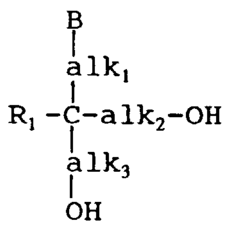

V

mit einer Verbindung der allgemeinen Formel PG-L, worin PG eine organische Schutzgruppe darstellt und L für eine organische Abgangsgruppe steht, umsetzt, wobei man eine Verbindung der allgemeinen Formel IV:

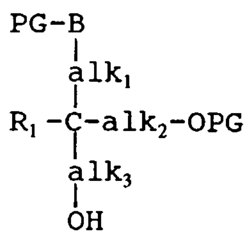

IV

erhält,
die so erhaltene Verbindung der Formel IV mit einem Metallhydrid behandelt und anschließend mit einem Phosphonatdiester der Formel VI

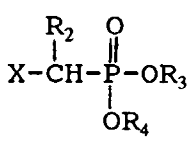

VI

umsetzt, wobei man eine Verbindung der allgemeinen Formel III

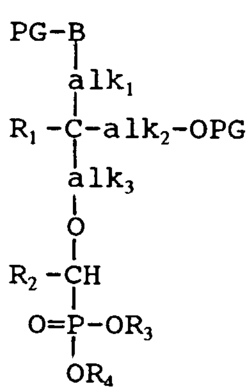

III

erhält,
die Schutzgruppe aus der so erhaltenen Verbindung der Formel III entfernt, wobei man eine Verbindung der Formel Ia, wie oben definiert, erhält, oder

b) ein Purin- oder Pyrimidinbase B mit einer Verbindung der allgemeinen Formel XII

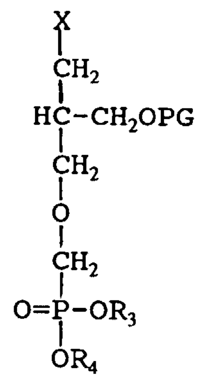

XII

umsetzt,
wobei man eine Verbindung der allgemeinen Formel

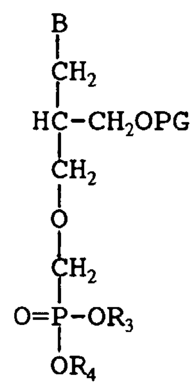

erhält;
die Schutzgruppe entfernt, wobei man eine Verbindung der allgemeinen Formel I'

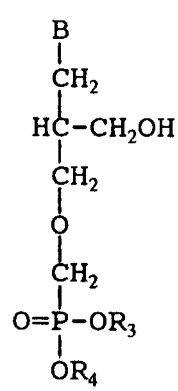

I'

erhält,

und, falls gewünscht, die so erhaltenen Diesterverbindungen der Formeln Ia und I' in die entsprechenden Monoester oder Disäureverbindungen umsetzt,
wobei in den vorhergehenden Formeln X, B, $alk_1$, $alk_2$, $alk_3$, $R_1$, $R_2$, $R_3$, $R_4$ und PG wie oben definiert sind.

**11.** Verfahren zur Herstellung des pharmazeutischen Mittels nach Anspruch 9, wobei man eine wirksame Menge mindestens einer Verbindung nach den Ansprüchen 1 bis 8 einem pharmazeutisch akzeptablen Träger einverleibt.

**Patentansprüche für folgenden Vertragsstaat : ES**

**1.** Verfahren zur Herstellung von Verbindungen der Formel I

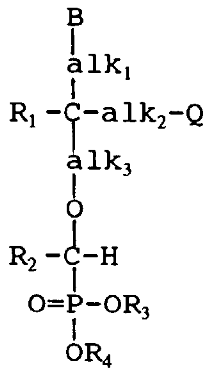

I

worin B für eine Purin- oder Pyrimidinbase, ausgewählt unter Xanthin, Hypoxanthin, Guanin, 8-Bromguanin, 8-Chlorguanin, 8-Aminoguanin, 8-Hydrazinoguanin, 8-Hydroxyguanin, 8-Methylguanin, 8-Thioguanin, 2-Aminopurin, 2,6-Diaminopurin, Cytosin, 5-Ethylcytosin, 5-Methylcytosin, Thymin, Uracil, 5-Bromuracil, 5-Joduracil, 5-Ethyluracil, 5-Propyluracil, 5-Vinyluracil und 5-Bromvinyl-uracil, steht;
$alk_1$, $alk_2$ und $alk_3$ unabhängig voneinander ausgewählt sind unter einer chemischen Bindung oder $C_1$-$C_4$-Alkylen;
Q für ein Wasserstoffatom und Hydroxyl steht;
$R_1$ und $R_2$ unabhängig voneinander ausgewählt sind unter einem Wasserstoffatom und $C_1$-$C_4$-Alkyl; und
$R_3$ und $R_4$ unabhängig voneinander ausgewählt sind unter einem Wasserstoffatom, $C_1$-$C_6$-Alkyl, Phenyl und Phenyl-$C_1$-$C_4$-alkylen;
und die entsprechenden Salze, Zwitterionen und/oder Solvate, ausgenommen diejenigen Verbindungen, worin $alk_1$ für Methylen, $R_1$ für ein Wasserstoffatom, $alk_2$ für eine chemische Bindung und Q für ein Wasserstoffatom stehen, $alk_3$ eine chemische Bindung ist, $R_2$ , $R_3$ und $R_4$ für ein Wasserstoffatom stehen und B für Uracil-1-yl, Thymin-1-yl, Cytosin-1-yl, Guanin-9-yl oder Hypoxanthin-9-yl steht,
und ausgenommen diejenigen Verbindungen, worin $alk_1$ für Methylen, $R_1$ für ein Wasserstoffatom, $alk_2$ für Methylen und Q für Hydroxyl steht, $alk_3$ eine chemische Bindung ist, $R_2$, $R_3$ und $R_4$ für ein Wasserstoffatom stehen und B für Uracil-1-yl, Cytosin-1-yl, Thymin-1-yl, Guanin-9-yl, Guanin-7-yl, Hypoxanthin-9-yl oder 2-Aminopurin-9-yl steht,
und ausgenommen (RS)-Verbindungen, worin $alk_1$ und $alk_2$ für Methylen stehen, $alk_3$ eine chemische Bindung ist, Q für Hydroxyl steht, $R_1$, $R_2$, $R_3$ und $R_4$ für ein Wasserstoffatom stehen und B für 2,6-Diaminopurin-9-yl steht,
und ausgenommen Verbindungen der Formel:

$$B\text{-}CH_2CH_2OCH_2P(O)(OC_2H_5)_2,$$

worin B für Xanthin, Hypoxanthin, Guanin, 2-Aminopurin-9-yl, 2,6-Diaminopurin-9-yl, Cytosin, Thymin oder Uracil steht,
wobei man

A) zur Herstellung der Verbindungen der Formel I, worin Q für ein Wasserstoffatom steht und $alk_2$ eine chemische Bindung ist, eine Purin- oder Pyrimidinbase B, die wie oben definiert sind, durch Behandlung mit einer

Base in ein Anion umwandelt;

das so erhaltene Anion der Purinbase B mit einem Phosphonatdiester der allgemeinen Formel II:

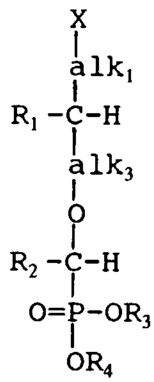

II

worin X eine organische Standardabgangsgruppe ist, $alk_1$, $alk_3$, $R_1$ und $R_2$ wie oben definiert sind; $R_3$ und $R_4$ wie oben definiert sind, aber kein Wasserstoffatom bedeuten, umsetzt, wobei man einen Phosphonatdiester der allgemeinen Formel Ia

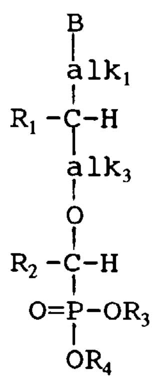

Ia

erhält, oder

B) zur Herstellung der Verbindungen der Formel I, worin

Q für Hydroxy steht,

a) eine Verbindung der allgemeinen Formel V

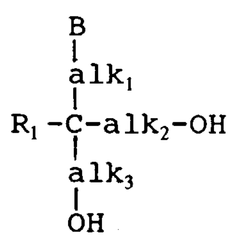

V

mit einer Verbindung der allgemeinen Formel PG-L, worin PG eine organische Schutzgruppe darstellt und L für eine organische Abgangsgruppe steht, umsetzt, wobei man eine Verbindung der allgemeinen Formel IV:

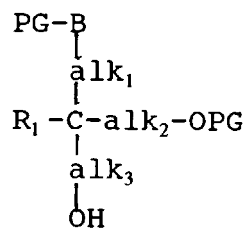

IV

erhält,

die so erhaltene Verbindung der Formel IV mit einem Metallhydrid behandelt und anschließend mit einem Phosphonatdiester der Formel VI

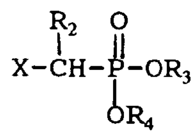

VI

umsetzt, wobei man eine Verbindung der allgemeinen Formel III

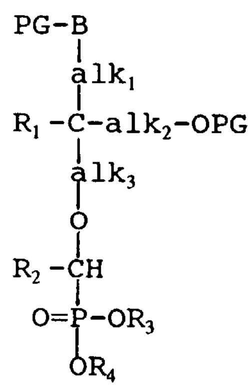

III

erhält,

die Schutzgruppe aus der so erhaltenen Verbindung der Formel III entfernt, wobei man eine Verbindung der Formel Ia, wie oben definiert, erhält, oder

b) ein Purin- oder Pyrimidinbase B mit einer Verbindung der allgemeinen Formel XII

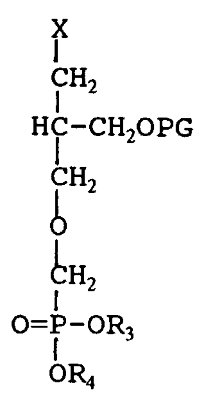

XII

umsetzt,
wobei man eine Verbindung der allgemeinen Formel

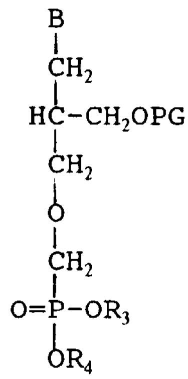

erhält;
die Schutzgruppe entfernt, wobei man eine Verbindung der allgemeinen Formel I'

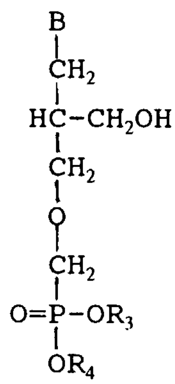

I'

erhält,

und, falls gewünscht, die so erhaltenen Diesterverbindungen der Formeln Ia und I' in die entsprechenden Monoester oder Disäureverbindungen umsetzt,
wobei in den vorhergehenden Formeln X, B, $alk_1$, $alk_2$, $alk_3$, $R_1$, $R_2$, $R_3$, $R_4$ und PG wie oben definiert sind.

**2.** Verfahren nach Anspruch 2, worin B für eine Purinbase steht, $R_1$, $R_2$ und Q für ein Wasserstoffatom stehen, $alk_1$ und $alk_2$ für Methylen stehen und $alk_3$ eine chemische Bindung ist.

**3.** Verfahren nach Anspruch 2, worin die Purinbase ein Guaninrest ist.

**4.** Verfahren nach Anspruch 1, worin B für eine Pyrimidinbase steht.

**5.** Verfahren nach Anspruch 2, worin $R_1$, $R_2$ und Q für ein Wasserstoffatom stehen.

**6.** Verfahren nach Anspruch 1, worin $R_3$ und $R_4$ für ein Wasserstoffatom stehen.

**7.** Verfahren nach Anspruch 1, worin einer der Reste $R_3$ und $R_4$ für ein Wasserstoffatom steht und der andere für $C_1$-$C_6$-Alkyl steht.

**8.** Verfahren nach Anspruch 1 zur Herstellung folgender Verbindungen:

9-(3-(Phosphonomethoxy)propyl)-guanin;
9-(4-(Phosphonomethoxy)butyl)-guanin;

8-Brom-9-(2-(phosphonomethoxy)ethyl)guanin;
1-(4-Phosphonomethoxy)-butyl)thymin;
9-(1-Methyl-2-(phosphonomethoxy)-ethyl)guanin;
9-(2-(Phosphonomethoxy)-l-propyl)guanin;
9-(2-Hydroxymethyl-3-(phosphonomethoxy)propyl)guanin;
8-Methyl-9-(2-(phosphonomethoxy)-ethyl)guanin;
9-(4-Hydroxy-3-(phosphonomethoxy)butyl)guanin;
9-(2-(Monoethylphosphonomethoxy)-ethyl)guanin;
9-(2-(Monomethylphosphonomethoxy)-ethyl)guanin;
9-(2-(Mono-n-propylphosphonomethoxy)ethyl)guanin;
9-(2-(Mono-isopropylphosphonomethoxy)ethyl)guanin;
9-(2-(1-Phosphono-1-ethoxy)ethyl)guanin;

**9.** Verfahren zur Herstellung eines pharmazeutischen Mittels, wobei man eine wirksame Menge mindestens einer Verbindung nach den Ansprüchen 1 bis 8 einem pharmazeutisch akzeptablen Träger einverleibt.

**Patentansprüche für folgenden Vertragsstaat : GR**

**1.** Verbindungen der Formel I

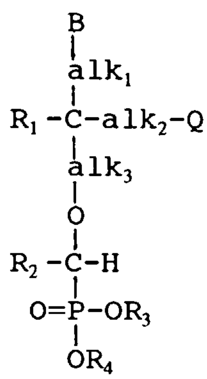

I

worin B für eine Purin- oder Pyrimidinbase, ausgewählt unter Xanthin, Hypoxanthin, Guanin, 8-Bromguanin, 8-Chlorguanin, 8-Aminoguanin, 8-Hydrazinoguanin, 8-Hydroxyguanin, 8-Methylguanin, 8-Thioguanin, 2-Aminopurin, 2,6-Diaminopurin, Cytosin, 5-Ethylcytosin, 5-Methylcytosin, Thymin, Uracil, 5-Bromuracil, 5-Joduracil, 5-Ethyluracil, 5-Propyluracil, 5-Vinyluracil und 5-Bromvinyl-uracil, steht;
$alk_1$, $alk_2$ und $alk_3$ unabhängig voneinander ausgewählt sind unter einer chemischen Bindung oder $C_1$-$C_4$-Alkylen;
Q für ein Wasserstoffatom und Hydroxyl steht;
$R_1$ und $R_2$ unabhängig voneinander ausgewählt sind unter einem Wasserstoffatom und $C_1$-$C_4$-Alkyl; und
$R_3$ und $R_4$ unabhängig voneinander ausgewählt sind unter einem Wasserstoffatom, $C_1$-$C_6$-Alkyl, Phenyl und Phenyl-$C_1$-$C_4$-alkylen;
und die entsprechenden Salze, Zwitterionen und/oder Solvate, ausgenommen diejenigen Verbindungen, worin $alk_1$ für Methylen, $R_1$ für ein Wasserstoffatom, $alk_2$ für eine chemische Bindung und Q für ein Wasserstoffatom stehen, $alk_3$ eine chemische Bindung ist, $R_2$ , $R_3$ und $R_4$ für ein Wasserstoffatom stehen und B für Uracil-1-yl, Thymin-1-yl, Cytosin-1-yl, Guanin-9-yl oder Hypoxanthin-9-yl steht,
und ausgenommen diejenigen Verbindungen, worin $alk_1$ für Methylen, $R_1$ für ein Wasserstoffatom, $alk_2$ für Methylen und Q für Hydroxyl steht, $alk_3$ eine chemische Bindung ist, $R_2$, $R_3$ und $R_4$ für ein Wasserstoffatom stehen und B für Uracil-1-yl, Cytosin-1-yl, Thymin-1-yl, Guanin-9-yl, Guanin-7-yl, Hypoxanthin-9-yl oder 2-Aminopurin-9-yl steht,
und ausgenommen (RS)-Verbindungen, worin $alk_1$ und $alk_2$ für Methylen stehen, $alk_3$ eine chemische Bindung ist, Q für Hydroxyl steht, $R_1$, $R_2$, $R_3$ und $R_4$ für ein Wasserstoffatom stehen und B für 2,6-Diaminopurin-9-yl steht,
und ausgenommen Verbindungen der Formel:

$$B\text{-}CH_2CH_2OCH_2P(O)(OC_2H_5)_2,$$

worin B für Xanthin, Hypoxanthin, Guanin, 2-Aminopurin-9-yl, 2,6-Diaminopurin-9-yl, Cytosin, Thymin oder Uracil steht.

**2.** Verbindungen nach Anspruch 1, worin B für eine Purinbase steht, $R_1$, $R_2$ und Q für ein Wasserstoffatom stehen, $alk_1$ und $alk_2$ für Methylen stehen und $alk_3$ eine chemische Bindung ist.

**3.** Verbindungen nach Anspruch 2, worin die Purinbase ein Guaninrest ist.

**4.** Verbindungen nach Anspruch 1, worin B für eine Pyrimidinbase steht.

**5.** Verbindungen nach Anspruch 2, worin $R_1$, $R_2$ und Q für ein Wasserstoffatom stehen.

**6.** Verbindungen nach Anspruch 1, worin $R_3$ und $R_4$ für ein Wasserstoffatom stehen.

**7.** Verbindungen nach Anspruch 1, worin einer der Reste $R_3$ und $R_4$ für ein Wasserstoffatom steht und der andere für $C_1$-$C_6$-Alkyl steht.

**8.** Verbindungen nach Anspruch 1, nämlich

9-(3-(Phosphonomethoxy)propyl)-guanin;
9-(4-(Phosphonomethoxy)butyl)-guanin;
8-Brom-9-(2-(phosphonomethoxy)ethyl)guanin;
1-(4-Phosphonomethoxy)-butyl)thymin;
9-(1-Methyl-2-(phosphonomethoxy)-ethyl)guanin;
9-(2-(Phosphonomethoxy)-1-propyl)guanin;
9-(2-Hydroxymethyl-3-(phosphonomethoxy)propyl)guanin;
8-Methyl-9-(2-(phosphonomethoxy)-ethyl)guanin;
9-(4-Hydroxy-3-(phosphonomethoxy)butyl)guanin;
9-(2-(Monoethylphosphonomethoxy)-ethyl)guanin;
9-(2-(Monomethylphosphonomethoxy)-ethyl)guanin;
9-(2-(Mono-n-propylphosphonomethoxy)ethyl)guanin;
9-(2-(Mono-isopropylphosphonomethoxy)ethyl)guanin;
9-(2-(1-Phosphono-1-ethoxy)ethyl)guanin.

**9.** Verfahren zur Herstellung eines pharmazeutischen Mittels wobei man eine wirksame Menge mindestens einer Verbindung nacl den Ansprüchen 1 bis 8 einem pharmazeutisch akzeptablen Träger einverleibt.

**10.** Verfahren zur Herstellung der Verbindungen nach Anspruch 1 bis 8, wobei man

A) zur Herstellung der Verbindungen der Formel I, worin

Q für ein Wasserstoffatom steht und $alk_2$ eine chemische Bindun ist, eine Purin- oder Pyrimidinbase B, die wie in Anspruch 1 definiert sind, durch Behandlung mit einer Base in ein Anion umwandelt;
das so erhaltene Anion der Purinbase B mit einem Phosphonatdiester der allgemeinen Formel II:

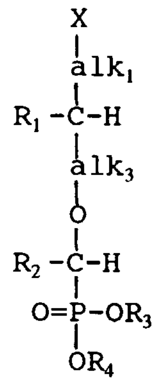

II

worin X eine organische Standardabgangsgruppe ist, $alk_1$, $alk_3$, $R_1$ und $R_2$ wie in Anspruch 1 definiert sind; $R_3$ und $R_4$ wie in Anspruch 1 definiert sind, aber kein Wasserstoffatom bedeuten, umsetzt, wobei man einen Phosphonatdiester der allgemeinen Formel Ia

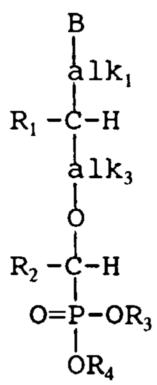

Ia

erhält, oder

B) zur Herstellung der Verbindungen der Formel I, worin

Q für Hydroxy steht,

a) eine Verbindung der allgemeinen Formel V

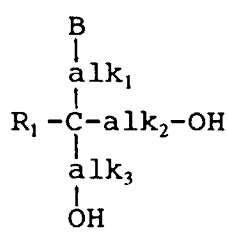

V

mit einer Verbindung der allgemeinen Formel PG-L, worin PG eine organische Schutzgruppe darstellt und L für eine organische Abgangsgruppe steht, umsetzt, wobei man eine Verbindung der allgemeinen Formel IV:

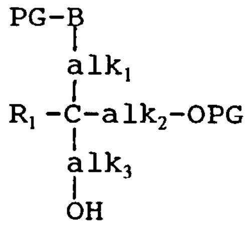

IV

erhält,
die so erhaltene Verbindung der Formel IV mit einem Metallhydrid behandelt und anschließend mit einem Phosphonatdiester der Formel VI

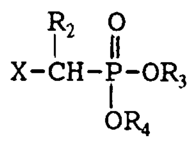

VI

umsetzt, wobei man eine Verbindung der allgemeinen Formel III

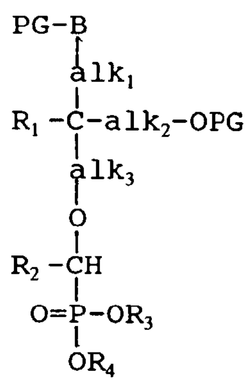

III

erhält,
die Schutzgruppe aus der so erhaltenen Verbindung der Formel III entfernt, wobei man eine Verbindung der Formel Ia, wie definiert, erhält, oder
b) ein Purin- oder Pyrimidinbase B mit einer Verbindung de allgemeinen Formel XII

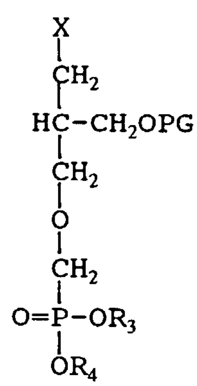

XII

umsetzt,
wobei man eine Verbindung der allgemeinen Formel

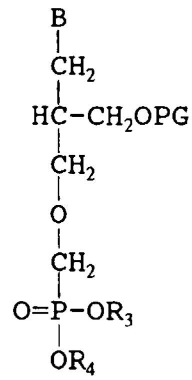

erhält;
die Schutzgruppe entfernt, wobei man eine Verbindung der allgemeinen Formel I'

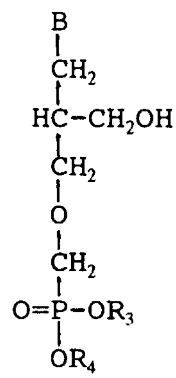

I'

erhält,

und, falls gewünscht, die so erhaltenen Diesterverbindungen der Formeln Ia und I' in die entsprechenden Monoester oder Disäureverbindungen umsetzt,
wobei in den vorhergehenden Formeln X, B, $alk_1$, $alk_2$, $alk_3$, $R_1$, $R_2$, $R_3$, $R_4$ und PG wie oben definiert sind.

## Revendications

**Revendications pour les Etats contractants suivants : AT, BE, CH, LI, DE, FR, GB, IT, LU, NL, SE**

1. Composé de formule I

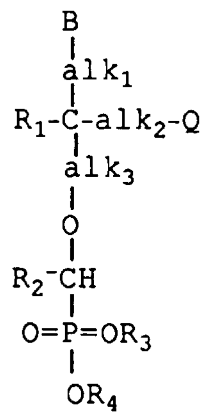

I

où B est une base de purine ou de pyrimidine choisie dans le groupe consistant en xanthine, hypoxanthine, guanine, 8-bromoguanine, 8-chloroguanine, 8-aminoguanine, 8-hydrazinoguanine, 8-hydroxyguanine, 8-méthylguanine, 8-thioguanine, 2-aminopurine, 2,6-diaminopurine, cytosine, 5-éthylcytosine, 5-méthylcytosine, thymine, uracil, 5-bromouracil, 5-iodouracil, 5-éthyluracil, 5-propyluracil, 5-vinyluracil et 5-bromovinyluracil;
$alk_1$, $alk_2$ et $alk_3$ sont indépendamment choisis parmi une liaison chimique ou un alkylène $C_1$-$C_4$;
Q est hydrogène et hydroxyle;
$R_1$ et $R_2$ sont indépendamment choisis parmi hydrogène et alkyle $C_1$-$C_4$; et
$R_3$ et $R_4$ sont indépendamment choisis parmi hydrogène, alkyle $C_1$-$C_6$, phényle et phényl-alkylène $C_1$-$C_4$; et les sels, zwitterions et/ou solvats correspondants, à l'exception des composés où $alk_1$ est méthylène, $R_1$ est hydrogène, $alk_2$ est une liaison chimique et Q est hydrogène, $alk_3$ est une liaison chimique, $R_2$, $R_3$ et $R_4$ sont hydrogène et B est uracil-1-yle, thymine-1-yle, cytosine-l-yle, guanine-9-yle ou hypoxanthine-9-yle et à l'exception des composés où $alk_1$ est méthylène, $R_1$ est hydrogène, $alk_2$ est méthylène et Q est hydroxyle, $alk_3$ est une liaison chimique, $R_2$, $R_3$ et $R_4$ sont hydrogène et B est uracil-1-yle, cytosine-1-yle, thymine-1-yle guanine-9-yle, guanine-7-yle, hypoxanthine-9-yle, ou 2-aminopurine-9-yle, et à l'exception des composés (RS) où $alk_1$ et $alk_2$ sont méthylène, $alk_3$ est une liaison chimique, Q est hydroxyle, $R_1$, $R_2$, $R_3$ et $R_4$ sont hydrogène et B est 2,6-diaminopurine-9-yle, et à l'exception des composés ayant pour formule :

$$B\text{-}CH_2CH_2OCH_2P(O)(OC_2H_5)_2$$

où B est xanthine, hypoxanthine, guanine, 2-amino-purine-9-yle, 2,6-diaminopurine-9-yle, cytosine, thymine ou uracil.

**2.** Composé de la revendication 1 où B est une base de purine, $R_1$ $R_2$, et Q sont hydrogène, $alk_1$ et $alk_2$ sont méthylène et $alk_3$ est une liaison chimique.

**3.** Composé de la revendication 2, où la base de purine est un fragment de guanine.

**4.** Composé de la revendication 1, où B est une base de pyrimidine.

**5.** Composé de la revendication 2, où $R_1$, $R_2$ et Q sont hydrogène.

**6.** Composé de la revendication 1, où $R_3$ ou $R_4$ sont hydrogène.

**7.** Composé de la revendication 1, où l'un de $R_3$ et $R_4$ est hydrogène et l'autre est alkyle $C_1$-$C_6$.

**8.** Composés de la revendication 1, c'est-à-dire

9-(3-(phosphonométhoxy)propyl)-guanine;
9-(4-(phosphonométhoxy)butyl)-guanine;

8-bromo-9-(2-(phosphono-méthoxy)éthyl)guanine;
1-(4-(phosphonométhoxy)-butyl)thymine;
9-(1-méthyl-2-(phosphonométhoxy)-éthyl)guanine;
9-(2-(phosphonométhoxy)-1-propyl)guanine;
9-(2-hydroxyméthyl-3-(phosphonométhoxy)propyl)guanine;
8-méthyl-9-(2-(phosphonométhoxy)-éthyl)guanine;
9-(4-hydroxy-3-(phosphonométhoxy)butyl)guanine;
9-(2-(monoéthylphosphonométhoxy)-éthyl)guanine;
9-(2-(monométhylphosphonométhoxy)-éthyl)guanine;
9-(2-mono-n-propylphosphonométhoxy)éthyl)guanine;
9-(2-(mono-isopropylphosphonométhoxy)éthyl)guanine;
9-(2-(1-phosphono-1-éthoxy)éthyl)guanine;

**9.** Composition pharmaceutique pour un usage antiviral comprenant une quantité antivirale efficace d'au moins un composé des revendications 1 à 8 en mélange avec un support acceptable en pharmacie.

**10.** Procédé de préparation des composés des revendications 1 à 8, qui comprend

A) pour la préparation des composés de formule I où Q est hydrogène et $alk_2$ est une liaison chimique, la conversion d'une base de purine ou de pyrimidine B qui est telle que définie à la revendication 1, en un anion, par traitement avec une base,
la réaction de l'anion ainsi obtenu de la base de purine B avec un diester de phosphonate de la formule générale II :

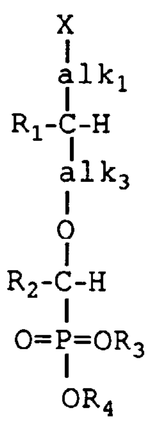

(II)

où X est un groupe partant organique standard;
$alk_1$, $alk_3$, $R_1$ et $R_2$ sont tels que définis à la revendication 1;
$R_3$ et $R_4$ sont tels que définis à la revendication 1, à l'exception que c'est de l'hydrogène, pour obtenir un diester de phosphonate de la formule générale la

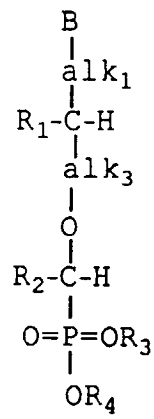

Ia

ou

B) pour la préparation des composés de formule I où Q est hydroxy

a) la réaction d'un composé de formule générale V

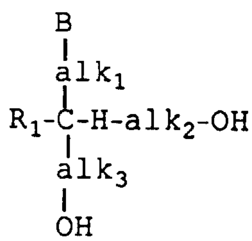

V

avec un composé de la formule générale PG-L, où PG représente un groupe organique protecteur et L est un groupe organique partant, pour obtenir un composé de la formule générale IV :

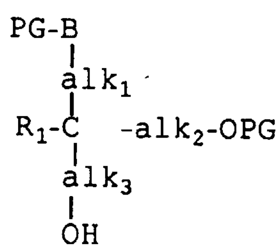

IV

le traitement du composé ainsi obtenu de formule IV avec un hydrure de métal avec ensuite réaction avec un diester de phosphonate de formule VI

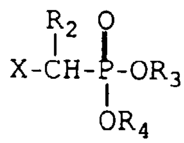

VI

pour obtenir un composé de formule générale III :

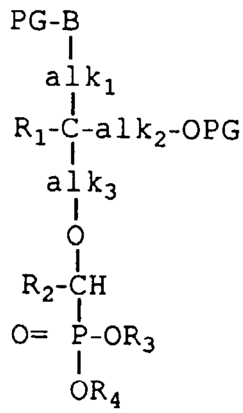

III

l'élimination des groupes protecteurs du composé ainsi obtenu de formule III pour obtenir un composé de la formule générale Ia telle que définie ci-dessus, ou

b) la réaction d'une base de purine ou de pyrimidine B avec un composé de la formule générale XII

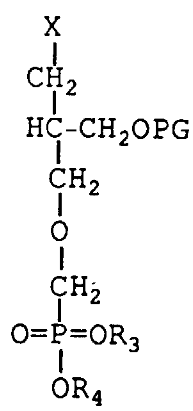

XII

pour obtenir un composé de la formule générale :

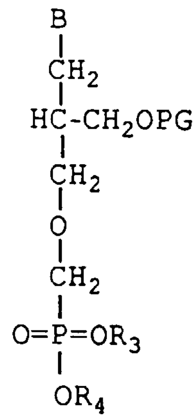

l'élimination du groupe protecteur pour obtenir un composé de la formule générale I'

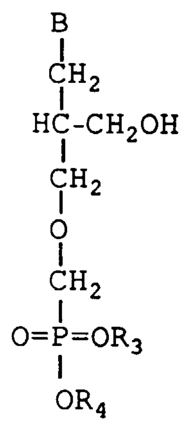

I'

et, si on le souhaite, la conversion des composés de diester ainsi obtenus des formules Ia et I' en composés de monoester ou diacide correspondants,

et dans les formules ci-dessus, X, B, $alk_1$, $alk_2$, $alk_3$, $R_1$, $R_2$, $R_3$, $R_4$ et PG sont tels que définis ci-dessus.

**11.** Procédé de préparation de la composition pharmaceutique de la revendication 9, qui comprend l'incorporation d'une quantité efficace d'au moins un composé des revendications 1 à 8 dans un support acceptable en pharmacie.

**Revendications pour l'Etat contractant suivant : ES**

**1.** Procédé pour la préparation d'un composé de formule I

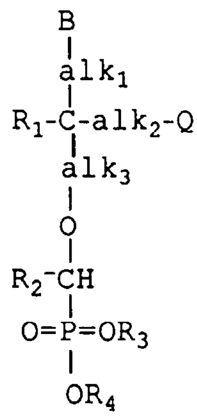

I

où B est une base de purine ou de pyrimidine choisie dans le groupe consistant en xanthine, hypoxanthine, guanine, 8-bromoguanine, 8-chloroguanine, 8-aminoguanine, 8-hydrazinoguanine, 8-hydroxyguanine, 8-méthylguanine, 8-thioguanine, 2-aminopurine, 2,6-diaminopurine, cytosine, 5-éthylcytosine, 5-méthylcytosine, thymine, uracil, 5-bromouracil, 5-iodouracil, 5-éthyluracil, 5-propyluracil, 5-vinyluracil et 5-bromovinyluracil;

$alk_1$, $alk_2$ et $alk_3$ sont indépendamment choisis parmi une liaison chimique ou un alkylène $C_1$-$C_4$;

Q est hydrogène et hydroxyle;

$R_1$ et $R_2$ sont indépendamment choisis parmi hydrogène et alkyle $C_1$-$C_4$; et

$R_3$ et $R_4$ sont indépendamment choisis parmi hydrogène, alkyle $C_1$-$C_6$, phényle et phényl-alkylène $C_1$-$C_4$; et les sels, zwitterions et/ou solvats correspondants, à l'exception des composés où $alk_1$ est méthylène, $R_1$ est hydrogène, $alk_2$ est une liaison chimique et Q est hydrogène, $alk_3$ est une liaison chimique, $R_2$, $R_3$ et $R_4$ sont hydrogène et B est uracil-1-yle, thymine-1-yle, cytosine-1-yle, guanine-9-yle ou hypoxanthine-9-yle et à l'exception des composés où $alk_1$ est méthylène, $R_1$ est hydrogène, $alk_2$ est méthylène et Q est hydroxyle, $alk_3$ est une liaison chimique, $R_2$, $R_3$ et $R_4$ sont hydrogène et B est uracil-1-yle, cytosine-1-yle, thymine-1-yle, guanine-9-yle, guanine-7-yle, hypoxanthine-9-yle, ou 2-aminopurine-9-yle, et à l'exception des composés (RS) où $alk_1$ et $alk_2$ sont méthylène, $alk_3$ est une liaison chimique, Q est hydroxyle, $R_1$, $R_2$, $R_3$ et $R_4$ sont hydrogène et B est 2,6-diaminopurine-9-yle, et à l'exception des composés ayant pour formule :

$$B\text{-}CH_2CH_2OCH_2P(O)(OC_2H_5)_2$$

où B est xanthine, hypoxanthine, guanine, 2-amino-purine-9-yle, 2,6-diaminopurine-9-yle, cytosine, thymine ou uracil,
qui comprend

A) pour la préparation des composés de formule I où

Q est hydrogène et $alk_2$ est une liaison chimique, la conversion d'une base de purine ou de pyrimidine B qui est telle que définie ci-dessus en un anion par traitement avec une base,

la réaction de l'anion ainsi obtenu de la base de purine B avec un diester de phosphonate de la formule générale II :

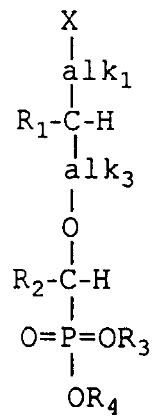

II

où X est un groupe partant organique standard;

$alk_1$, $alk_3$, $R_1$ et $R_2$ sont tels que définis ci-dessus;

$R_3$ et $R_4$ sont tels que définis ci-dessus à l'exception qu'ils sont de l'hydrogène, pour obtenir un diester de phosphonate de la formule générale la

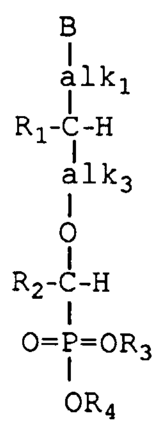

(Ia)

ou

B) pour la préparation des composés de formule I où

Q est hydroxy

a) la réaction d'un composé de la formule générale V

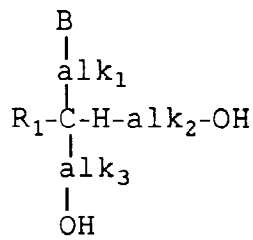

V

avec un composé de la formule PG-L, où PG représente un groupe protecteur organique et L est un groupe partant organique pour obtenir un composé de la formule générale IV :

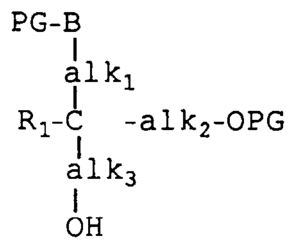

IV

le traitement du composé ainsi obtenu de formule IV avec un hydrure de métal, avec ensuite réaction avec un diester de phosphonate de formule VI

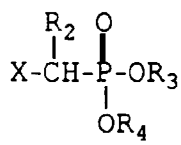

VI

pour obtenir un composé de la formule générale III:

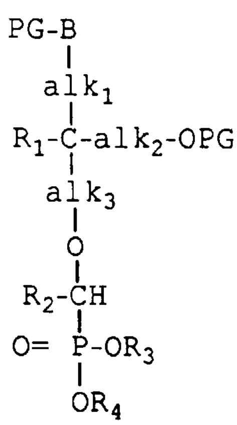

III

l'élimination des groupes protecteurs du composé ainsi obtenu de formule III pour obtenir un composé de la formule générale Ia tel que défini ci-dessus, ou

b) la réaction d'une base de purine ou de pyrimidine B avec un composé de la formule générale XII

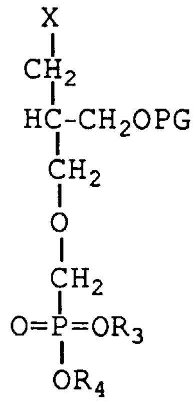

(XII)

pour obtenir un composé de la formule générale :

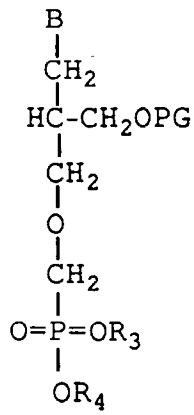

l'élimination du groupe protecteur pour obtenir un composé de la formule générale I'

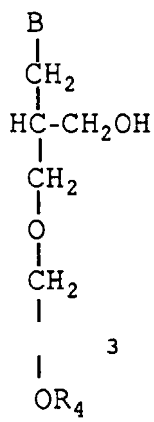

I'

et, si on le souhaite, la conversion des composés de diester ainsi obtenus des formules Ia et I' en composés

correspondants de monoester ou de diacide,
dans les formules ci-dessus X, B, $alk_1$, $alk_2$, $alk_3$, $R_1$, $R_2$, $R_3$, $R_4$ et PG sont tels que définis ci-dessus.

**2.** Procédé de la revendication 1 où B est une base de purine, $R_1$, $R_2$ et Q sont de l'hydrogène, $alk_1$ et $alk_2$ sont méthylène et $alk_3$ est une liaison chimique.

**3.** Procédé selon la revendication 2 où la base de purine est un fragment de guanine.

**4.** Procédé selon la revendication 1 où B est une base pyrimidine.

**5.** Procédé selon la revendication 2 où $R_1$, $R_2$ et Q sont de l'hydrogène.

**6.** Procédé selon la revendication 1 où $R_3$ et $R_4$ sont de l'hydrogène.

**7.** Procédé selon la revendication 1 où l'un de $R_3$ et $R_4$ est de l'hydrogène et l'autre est alkyle $C_1$-$C_6$.

**8.** Procédé selon la revendication 1 pour la préparation des composés suivants :

9-(3-(phosphonométhoxy)propyl)-guanine;
9-(4-(phosphonométhoxy)butyl)-guanine;
8-bromo-9-(2-(phosphono-méthoxy)éthyl)guanine;
1-(4-(phosphonométhoxy)-butyl)thymine;
9-(1-méthyl-2-(phosphonométhoxy)-éthyl)guanine;
9-(2-(phosphonométhoxy)-1-propyl)guanine;
9-(2-hydroxyméthyl-3-(phosphonométhoxy)propyl)guanine;
8-méthyl-9-(2-(phosphonométhoxy)-éthyl)quanine;
9-(4-hydroxy-3-(phosphonométhoxy)butyl)guanine;
9-(2-(monoéthylphosphonométhoxy)-éthyl)guanine;
9-(2-(monométhylphosphonométhoxy)-éthyl)guanine;
9-(2-mono-n-propylphosphonométhoxy)éthyl)guanine;
9-(2-(mono-isopropylphosphonométhoxy)éthyl)guanine;
9-(2-(1-phosphono-1-éthoxy)éthyl)guanine;

**9.** Procédé pour la préparation d'une composition pharmaceutique qui comprend l'incorporation d'une quantité efficace d'au moins un composé tel que défini aux revendications 1 à 8 dans un support pharmaceutiquement acceptable.

**Revendications pour l'Etat contractant suivant : GR**

**1.** Composé de formule I

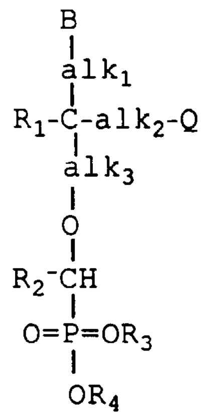

I

où B est une base de purine ou de pyrimidine choisie dans le groupe consistant en xanthine, hypoxanthine, guanine, 8-bromoguanine, 8-chloroguanine, 8-aminoguanine, 8-hydrazinoguanine, 8-hydroxyguanine, 8-méthylguanine, 8-thioguanine, 2-aminopurine, 2,6-diaminopurine, cytosine, 5-éthylcytosine, 5-méthylcytosine, thymine, uracil, 5-bromouracil, 5-iodouracil, 5-éthyluracil, 5-propyluracil, 5-vinyluracil et 5-bromovinyluracil;

$alk_1$, $alk_2$ et $alk_3$ sont indépendamment choisis parmi une liaison chimique ou un alkylène $C_1$-$C_4$;

Q est hydrogène et hydroxyle;.

$R_1$ et $R_2$ sont indépendamment choisis parmi hydrogène et alkyle $C_1$-$C_4$; et

$R_3$ et $R_4$ sont indépendamment choisis parmi hydrogène, alkyle $C_1$-$C_6$, phényle et phényl-alkylène $C_1$-$C_4$; et les sels, zwitterions et/ou solvats correspondants, à l'exception des composés où $alk_1$ est méthylène, $R_1$ est hydrogène, $alk_2$ est une liaison chimique et Q est hydrogène, $alk_3$ est une liaison chimique, $R_2$, $R_3$ et $R_4$ sont hydrogène et B est uracil-1-yle, thymine-1-yle, cytosine-1-yle, guanine-9-yle ou hypoxanthine-9-yle et à l'exception des composés où $alk_1$ est méthylène, $R_1$ est hydrogène, $alk_2$ est méthylène et Q est hydroxyle, $alk_3$ est une liaison chimique, $R_2$, $R_3$ et $R_4$ sont hydrogène et B est uracil-1-yle, cytosine-1-yle, thymine-1-yle guanine-9-yle, guanine-7-yle, hypoxanthine-9-yle, ou 2-aminopurine-9-yle, et à l'exception des composés (RS) où $alk_1$ et $alk_2$ sont méthylène, $alk_3$ est une liaison chimique, Q est hydroxyle, $R_1$, $R_2$, $R_3$ et $R_4$ sont hydrogène et B est 2,6-diaminopurine-9-yle, et à l'exception des composés ayant pour formule :

$$B\text{-}CH_2CH_2OCH_2P(O)(OC_2H_5)_2$$

où B est xanthine, hypoxanthine, guanine, 2-amino-purine-9-yle, 2,6-diaminopurine-9-yle, cytosine, thymine ou uracil.

**2.** Composé de la revendication 1 où B est une base de purine, $R_1$ $R_2$, et Q sont hydrogène, $alk_1$ et $alk_2$ sont méthylène et $alk_3$ est une liaison chimique.

**3.** Composé de la revendication 2, où la base de purine est un fragment de guanine.

**4.** Composé de la revendication 1, où B est une base de pyrimidine.

**5.** Composé de la revendication 2, où $R_1$, $R_2$ et Q sont hydrogène.

**6.** Composé de la revendication 1, où $R_3$ ou $R_4$ sont hydrogène.

**7.** Composé de la revendication 1, où l'un de $R_3$ et $R_4$ est hydrogène et l'autre est alkyle $C_1$-$C_6$.

**8.** Composés de la revendication 1, c'est-à-dire

9-(3-(phosphonométhoxy)propyl)-guanine;
9-(4-(phosphonométhoxy)butyl)-guanine;
8-bromo-9-(2-(phosphono-méthoxy)éthyl)guanine;
1-(4-(phosphonométhoxy)-butyl)thymine;
9-(1-méthyl-2-(phosphonométhoxy)-éthyl)guanine;
9-(2-(phosphonométhoxy)-1-propyl)guanine;
9-(2-hydroxyméthyl-3-(phosphonométhoxy)propyl)-guanine;
8-méthyl-9-(2-(phosphonométhoxy)-éthyl)guanine;
9-(4-hydroxy-3-(phosphonométhoxy)butyl)guanine;
9-(2-(monoéthylphosphonométhoxy)-éthyl)guanine;
9-(2-(monométhylphosphonométhoxy)-éthyl)guanine;
9-(2-mono-n-propylphosphonométhoxy)éthyl)guanine;
9-(2-(mono-isopropylphosphonométhoxy)éthyl)guanine;
9-(2-(1-phosphono-1-éthoxy)éthyl)guanine;

**9.** Procédé de préparation d'une composition pharmaceutique qui comprend l'incorporation d'une quantité efficace d'au moins un composé tel que défini aux revendications 1 à 8 dans un support pharmaceutiquement acceptable.

**10.** Procédé de préparation des composés des revendications 1 à 8, qui comprend

A) pour la préparation des composés de formule I où Q est hydrogène et $alk_2$ est une liaison chimique, la conversion d'une base de purine ou de pyrimidine B qui est telle que définie à la revendication 1, en un anion, par traitement avec une base,

la réaction de l'anion ainsi obtenu de la base de purine B avec un diester de phosphonate de la formule générale II :

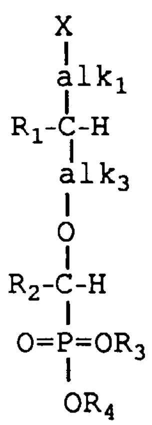

(II)

où X est un groupe partant organique standard;

$alk_1$, $alk_3$, $R_1$ et $R_2$ sont tels que définis à la revendication 1;

$R_3$ et $R_4$ sont tels que définis à la revendication 1, à l'exception que c'est de l'hydrogène, pour obtenir un diester de phosphonate de la formule générale Ia

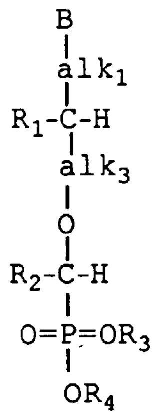

Ia

ou

B) pour la préparation des composés de formule I où Q est hydroxy

a) la réaction d'un composé de formule générale V

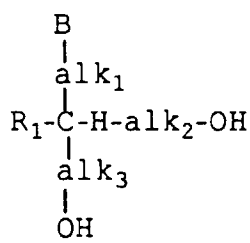

V

avec un composé de la formule générale PG-L, où PG représente un groupe organique protecteur et L est un groupe organique partant, pour obtenir un composé de la formule générale IV :

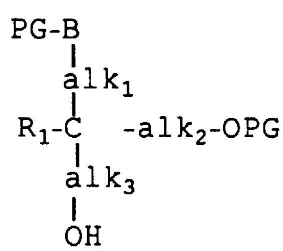

IV

le traitement du composé ainsi obtenu de formule IV avec un hydrure de métal avec ensuite réaction avec un diester de phosphonate de formule VI

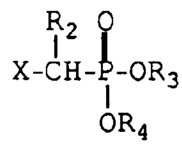

VI

pour obtenir un composé de formule générale III :

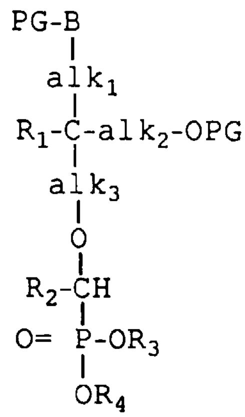

III

l'élimination des groupes protecteurs du composé ainsi obtenu de formule III pour obtenir un composé de la formule générale la telle que définie ci-dessus, ou

b) la réaction d'une base de purine ou de pyrimidine B avec un composé de la formule générale XII

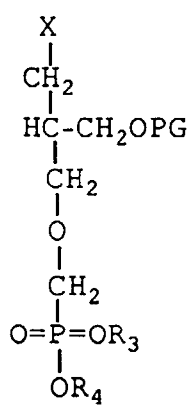

XII

pour obtenir un composé de la formule générale :

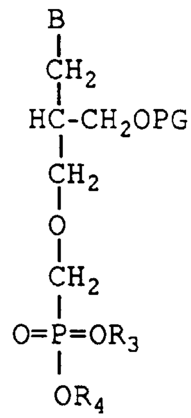

l'élimination du groupe protecteur pour obtenir un composé de la formule générale I'

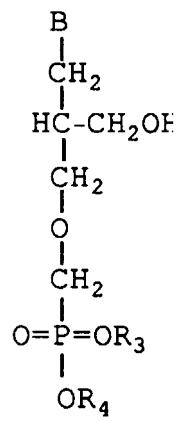

I'

et, si on le souhaite, la conversion des composés de diester ainsi obtenus des formules Ia et I' en composés de monoester ou diacide correspondants,
et dans les formules ci-dessus, X, B, $alk_1$, $alk_2$, $alk_3$, $R_1$, $R_2$, $R_3$, $R_4$ et PG sont tels que définis ci-dessus.